# EUROPÄISCHE PATENTSCHRIFT

(11) **EP 2 120 923 B1**
(45) Veröffentlichungstag und Bekanntmachung des Hinweises auf die Patenterteilung: **18.07.2012**
(21) Anmeldenummer: 08700981.7
(22) Anmeldetag: 04.01.2008
(51) Int. Cl.: A61K 31/351, A61P 3/00

(54) **VERWENDUNG VON SUBSTITUIERTEN PYRANONSÄUREDERIVATEN ZUR BEHANDLUNG DES METABOLISCHEN SYNDROMS**
USE OF SUBSTITUTED PYRANONE ACID DERIVATIVES FOR THE TREATMENT OF METABOLIC SYNDROME
UTILISATION DE DÉRIVÉS ACIDES DE PYRANONE SUBSTITUÉS DANS LE TRAITEMENT DU SYNDROME MÉTABOLIQUE

(30) Priorität: 16.01.2007 DE 102007002260
(43) Veröffentlichungstag der Anmeldung: 25.11.2009
(73) Patentinhaber: SANOFI, 75008 Paris (FR)
(72) Erfinder: BRUMMERHOP, Harm, 65926 Frankfurt am Main (DE); STENGELIN, Siegfried, 65926 Frankfurt am Main (DE); HEUER, Hubert, 65926 Frankfurt am Main (DE); KILP, Susanne, 55452 Hergenfeld (DE); HERLING, Andreas, 65926 Frankfurt am Main (DE); KLABUNDE, Thomas, 65926 Frankfurt am Main (DE); KADEREIT, Dieter, 65926 Frankfurt am Main (DE); URMANN, Matthias, 65926 Frankfurt am Main (DE)
(74) Vertreter: Then, Johann
(86) Internationale Anmeldenummer: PCT/EP2008/000029
(87) Internationale Veröffentlichungsnummer: WO 2008/086949

(56) Entgegenhaltungen:
- EP-A- 0 171 814
- US-A- 3 152 148
- US-B1- 6 552 073

## Beschreibung

Die Erfindung betrifft die Verwendung von substituierten Pyranonsäurederivaten sowie derer physiologisch verträglichen Salze zur Herstellung von Medikamenten zur Behandlung des Metabolischen Syndroms.

In US 6,552,073 sind strukturähnliche Pyranonsäurederivaten beschrieben, die eine antiproliferative Wirkung zeigen. US 3,152,148 und EP 0 171 814 zeigen strukturähnliche Verbindungen.

Der Erfindung lag die Aufgabe zugrunde, Verbindungen zur Verfügung zu stellen, die zur Behandlung des Metabolischen Syndroms verwendet werden können und die insbesonders eine therapeutisch verwertbare lipidsenkende Wirkung entfalten. Weiter bevorzugt sollten sie zur Behandlung der Diabetischen Dislipidämie geeignet sein.Weiter bevorzugt sollte eine Senkung der freien Fettsäuren (FFA), von Glycerol und der Triglyceride im Plasma erreicht werden.

Die Erfindung betrifft daher die Verwendung der Verbindungen der Formel I, 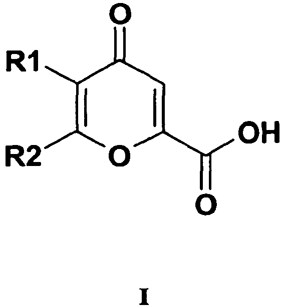 worin bedeuten
- R1: H, F, Cl, Br, I, OH, CF₃, NO₂, CN, OCF₃, NH₂, NH(C₁-C₆)-Alkyl, N((C₁-C₆)-Alkyl)₂, SF₅, SO₂-CH₃, COOH, COO-(C₁-C₆)-Alkyl, CONH₂, (C₁-C₂₀)-Alkyl, (C₃-C₂₀)-Cycloalkyl, (C₂-C₂₀)-Alkenyl, (C₂-C₂₀)-Alkinyl, Aryl, Heterozyklus, wobei in den (C₁-C₂₀)-Alkyl, (C₂-C₂₀)-Alkenylresten ein oder mehrere einzelne -CH₂- oder-CH- Gruppen durch -0- ersetzt sein können und wobei die Alkyl-, Cycloalkyl-, Alkenyl-, Alkinyl-, Aryl- und Heterozyklylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl; PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl)-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
- R2: H, F, Cl, Br, I, OH, CF₃, NO₂, CN, OCF₃, NH₂, NH(C₁-C₆)-Alkyl, N((C₁-C₆)-Alkyl)₂, SF₅, SO₂-CH₃, COOH, COO-(C₁-C₆)-Alkyl, CONH₂, (C₁-C₂₀)-Alkyl, (C₃-C₂₀)-Cycloalkyl, (C₂-C₂₀)-Alkenyl, (C₂-C₂₀)-Alkinyl, Aryl, Heterozyklus, wobei in den (C₁-C₂₀)-Alkyl, (C₂-C₂₀)-Alkenylresten ein oder mehrere einzelne -CH₂- oder-CH- Gruppen durch -0- ersetzt sein können und wobei die Alkyl-, Cycloalkyl-, Alkenyl-, Alkinyl-, Aryl- und Heterozyklylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl; PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
- oder R1 und R2: bilden gemeinsam einen 3 bis 8 gliedrigen Aryl-, Cycloalkyl- oder Heterozyklyl-Ring, wobei der Aryl-, Cycloalkyl- oder Heterozyklyl-Ring substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂ und wobei der Aryl-, Cycloalkyl- oder Heterozyklyl-Ring mit einem weiteren Aryl-, Cycloalkyl- oder Heterozyklyl-Ring anneliert sein kann;
sowie derer physiologisch verträglichen Salze.

Bevorzugt ist die Verwendung der Verbindungen der Formel I, worin bedeuten
- R1: H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch - O- ersetzt sein können und wobei die Alkyl-, Alkenyl- und Arylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl; PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO2-N((CH₂)ₙ-Aryl)₂, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl)-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)_{2,} N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
- R2: H, OH, COOH, (C₁-C₈)-Alkyl, (C₃-C₈)-Cycloalkyl, (C₂-C₈)-Alkenyl, wobei in den (C₁-C₈)-Alkyl, oder (C₂-C₈)-Alkenylresten ein oder mehrere einzelne -CH₂- oder - CH- Gruppen durch -0- ersetzt sein können und wobei die Alkyl-, Cycloalkyl und Alkenylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂N((C₁-C₆)-Alkyl)(CH₂)ₙ-Heterozyklyl, SO₂-N((CH₂)₂-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- oder R1 und R2: bilden gemeinsam einen 3 bis 8 gliedrigen Aryl-, Cycloalkyl- oder Heterozyklyl-Ring, wobei der Aryl-, Cycloalkyl- oder Heterozyklyl-Ring substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
sowie derer physiologisch verträglichen Salze.

Besonders bevorzugt ist die Verwendung der Verbindungen der Formel I, worin bedeuten
- R1: H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch - O- ersetzt sein können und wobei die Alkyl-, Alkenyl- und Arylreste ein oder mehrfach substituiert sein können mit
=O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- R2: H, (C₁-C₈)-Alkyl, (C₂-C₈)-Alkenyl;
sowie derer physiologisch verträglichen Salze.

Ganz besonders bevorzugt ist die Verwendung der Verbindungen der Formel I, worin bedeuten
- R1: H, OH, (C₁-C₈)-Alkyl, (C₂-C₄)-Alkenyl, wobei in den (C₁-C₈)-Alkyl, (C₂-C₄)-Alkenylresten ein oder mehrere einzelne -CH₂- oder -CH- Gruppen durch -Oersetzt sein können und wobei die Alkyl- und Alkenylreste ein oder mehrfach substituiert sein können mit
=O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- R2: H, (C₁-C₈)-Alkyl, (C₂-C₄)-Alkenyl;
sowie derer physiologisch verträglichen Salze.

Weiter bevorzugt ist die Verwendung der Verbindungen der Formel I, worin bedeuten
- R1: (C₁-C₈)-Alkyl, (C₂-C₄)-Alkenyl, wobei in den (C₁-C₈)-Alkyl, (C₂-C₄)-Alkenylresten ein oder mehrere einzelne -CH₂- oder -CH- Gruppen durch -0- ersetzt sein können und wobei die Alkyl- und Alkenylreste ein oder mehrfach substituiert sein können mit
=O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- R2: H, (C₁-C₈)-Alkyl, (C₂-C₄)-Alkenyl;
sowie derer physiologisch verträglichen Salze.

Weiter bevorzugt ist die Verwendung der Verbindungen der Formel I, worin bedeuten
- R1: (C₂-C₈)-Alkyl, wobei der Alkylrest ein oder mehrfach mit F substituiert ist;
- R2: H, (C₁-C₈)-Alkyl, (C₂-C₄)-Alkenyl;
sowie derer physiologisch verträglichen Salze.

Weiter bevorzugt ist die Verwendung der Verbindungen der Formel I, worin bedeuten
- R1: H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch - O- ersetzt sein können und wobei die Alkyl-, Alkenyl- und Arylreste ein oder mehrfach substituiert sein können mit
F, =O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- R2: H, (C₁-C₈)-Alkyl, (C₂-C₈)-Alkenyl;
sowie derer physiologisch verträglichen Salze zur Herstellung eines Medikaments zur Behandlung des Metabolischen Syndroms.

Weiterhin bevorzugt ist die Verwendung der Verbindungen der Formel I, worin bedeuten
- R1: H, OH, (C₁-C₈)-Alkyl, (C₂-C₄)-Alkenyl, wobei in den (C₁-C₈)-Alkyl, (C₂-C₄)-Alkenylresten ein oder mehrere einzelne -CH₂- oder -CH- Gruppen durch -0-ersetzt sein können und wobei die Alkyl- und Alkenylreste ein oder mehrfach substituiert sein können mit
F, =O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
- R2: H, (C₁-C₈)-Alkyl, (C₂-C₄)-Alkenyl;
sowie derer physiologisch verträglichen Salze zur Herstellung eines Medikaments zur Behandlung des Metabolischen Syndroms.

Die Erfindung betrifft weiterhin neue Verbindungen der Formel I, worin bedeuten 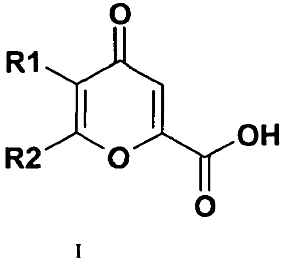
- R1: (C₁-C₈)-Alkyl, (C₂-C₄)-Alkenyl, wobei die Alkyl- und Alkenylreste ein oder mehrfach mit F substituiert sein können;
- R2: H;
sowie derer physiologisch verträglichen Salze.

Besonders bevorzugt sind Verbindungen der Formel I, worin bedeuten
- R1: (C₂-C₈)-Alkyl, wobei der Alkylrest ein oder mehrfach mit F substituiert sein kann;
- R2: H;
sowie derer physiologisch verträglichen Salze.

In einer Ausführungsform sind Verbindungen der Formel I bevorzugt, worin R1 gleich (C₂-C₈)-Alkyl, wobei der Alkylrest ein oder mehrfach mit F substituiert sein kann, ist.

In einer Ausführungsform sind Verbindungen der Formel I bevorzugt, worin R1 gleich (C₂-C₈)-Alkyl ist.

In einer Ausführungsform sind Verbindungen der Formel I bevorzugt, worin R1 gleich O-(C₂-C₈)-Alkyl ist, wobei der Alkylrest ein oder mehrfach mit F substituiert sein kann.

In einer Ausführungsform sind Verbindungen der Formel I bevorzugt, worin R1 gleich (C₂-C₄)-Alkenyl ist.

In einer Ausführungsform sind Verbindungen der Formel I bevorzugt, worin R1 gleich (C₂-C₄)-Alkenyl-Phenyl ist, wobei der Phenylrest mit F, Cl, Br, I, CF₃, OCF₃, NO₂, N₃, CN, (C₁-C₆)Alkyl oder O(C₁-C₆)Alkyl substituiert sein kann.

In einer Ausführungsform sind Verbindungen der Formel I bevorzugt, worin R1 gleich O-Benzyl ist, wobei der Benzylrest mit F, Cl, Br, I, CF₃, OCF₃, NO₂, N₃, CN, (C₁-C₆)Alkyl oder O(C₁-C₆)Alkyl substituiert sein kann.

In einer Ausführungsform sind Verbindungen der Formel I bevorzugt, worin R1 gleich O-Phenyl ist, wobei der Phenylrest mit F, Cl, Br, I, CF₃, OCF₃, NO₂, N₃, CN, (C₁-C₆)Alkyl oder O(C₁-C₆)Alkyl substituiert sein kann.

In einer Ausführungsform sind Verbindungen der Formel I bevorzugt, worin R1 gleich Phenyl ist, wobei der Phenylrest mit F, Cl, Br, I, CF₃, OCF₃, NO₂, N₃, CN, (C₁-C₆)Alkyl oder O(C₁-C₆)Alkyl substituiert sein kann.

In einer Ausführungsform sind Verbindungen der Formel I bevorzugt, worin R2 gleich H ist.

In einer Ausführungsform sind Verbindungen der Formel I bevorzugt, worin R2 gleich (C₁-C₈)-Alkyl ist.

In einer Ausführungsform sind Verbindungen der Formel I bevorzugt, worin R2 gleich (C₂-C₄)-Alkenyl ist.

Können Reste oder Substituenten mehrfach in den Verbindungen der Formel I auftreten, so können sie alle unabhängig voneinander die angegebenen Bedeutungen haben und gleich oder verschieden sein.

Die Erfindung bezieht sich auf die Verwendung der Verbindungen der Formel I, in Form ihrer Racemate, racemischen Mischungen und reinen Enantiomere sowie auf ihre Diastereomere und Mischungen davon.

Die Erfindung bezieht sich weiterhin auf die Verwendung der Verbindung der Formel I, worin R1 und R2 gleich Wasserstoff sind, als Arzneimittel.

Unter einem Alkylrest wird eine geradkettige oder verzweigte Kohlenwasserstoffkette mit einem oder mehreren Kohlenstoffen verstanden, wie z.B. Methyl, Ethyl, Propyl, Butyl, Hexyl, Isopropyl, Isobutyl, Neopentyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, Aryl, Heterozyklyl, O-(C₁-C₆)-Alkyl, O-COO-(C₁-C₆)-Alkyl, O-CO-(C₁-C₆)-Alkyl, O-CO-(C₁-C₆)-Aryl, O-CO-(C₁-C₆)-Heterozyklyl, PO₃H₂, P(O)(OAlkyl)2, (C₁-C₆)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N[((C₁-C₆)-Alkyl)(CH₂)ₙ-Aryl], SO₂-N[((C₁-C₆)-Alkyl)(CH₂)ₙ-Heterozyklyl], SO₂-N((CH₂)ₙ-Aryl)₂, , SO₂-N((CH₂)ₙ-(Heterozyklyl)₂, wobei n = 0 - 6 sein kann und der Aryl- oder Heterozyklyl- Rest bis zu dreifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.
Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, Aryl, Heterozyklyl, O-(C₁-C₆)-Alkyl O-CO-(C₁-C₆)-Alkyl, O-CO-(C₁-C₆)-Aryl, O-CO-(C₁-C₆)-Heterozyklyl,;

PO₃H₂, P(O)(OAlkyl)2, (C₁-C₆)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂; NH(C₁-C₆)-Alkyl, N((C₁-C₆)-Alkyl)₂, SF₅, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)2, (C1-C6)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.
Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, SF₅, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,;
PO₃H₂, P(O)(OAlkyl)2, (C1-C6)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, NO₂, SF₅, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N-((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,; PO₃H₂, P(O)(OAlkyl)2, (C1-C6)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter Heterozyklus, Heterozyklyl bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterozyklische Rest mit Benzolkernen kondensiert ist.

Geeignete Heterozyklyl- bzw. "heterozyklische Reste" sind Acridinyl, Benzimidazolyl, Benzofuryl, Benzothienyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazol, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl, Aziridinyl, Azetininyl, Azepanyl, Azocanyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterozyklen.

Die heterozyklischen Ringe bzw. Heterozyklische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)2, (C1-C6)-Alkylen-P(O)(OAlkyl)2, O-P(O)(OH)_{2,} O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N(C₁-C₆)-Alkyl)(CH₂)ₙ-Aryl, SO₂-N(C₁-C₆)-Alkyl)(CH₂)ₙ-Heterozyklyl, SO₂-N((CH₂)ₙ-Aryl)₂, SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF5, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, SF5, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure ferner L-Ascorbinsäure, Salizylsäure, 1,2-Benzisothiazol-3(2H)-on und 6-Methyl-1,2,3-oxathiazin-4(3H)-on-2,2-dioxid. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Verbindung(en) der Formel (I) können auch in Kombination mit weiteren Wirkstoff verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2006, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulfonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin
verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, PCT/EP 2003/05815, PCT/EP 2003/05814, PCT/EP 2003/05816, EP 0114531, US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, G1 262570, verabreicht.

Bei einer Ausführungsform der Erfindung werden die Verbindungen der Formel I in Kombination mit PPAR alfa Agonist, wie z.B. GW 9578, GW 7647, verabreicht.

Bei einer Ausführungsform der Erfindung werden die Verbindungen der Formel I in Kombination mit einem gemischten PPAR alfa/gamma Agonisten, wie z.B. GW 1536, AVE 8042, AVE 8134, AVE 0847, oder wie in PCT/US 2000/11833, PCT/US 2000/11490, WO 03/020269 beschrieben, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulfonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxi]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Adenosin A1 Agonisten, wie z. B. solchen, die in EP 0912520 oder PCT/EP06749beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxi)-ethylamino]-ethanol Hydrochlorid (WO 01/8/834541)), CB1 (Cannabinoid Rezeptor 1) Rezeptor Antagonisten (zB. Rimonabant oder die in WO 02/28346 genannten Wirkstoffe, MSH (Melanocyt-stimulierendes Hormon)-Agonisten, CCK-A Agonisten (z.B. {2-[4-(4-Chloro-2,5-dimethoxi-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxi-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 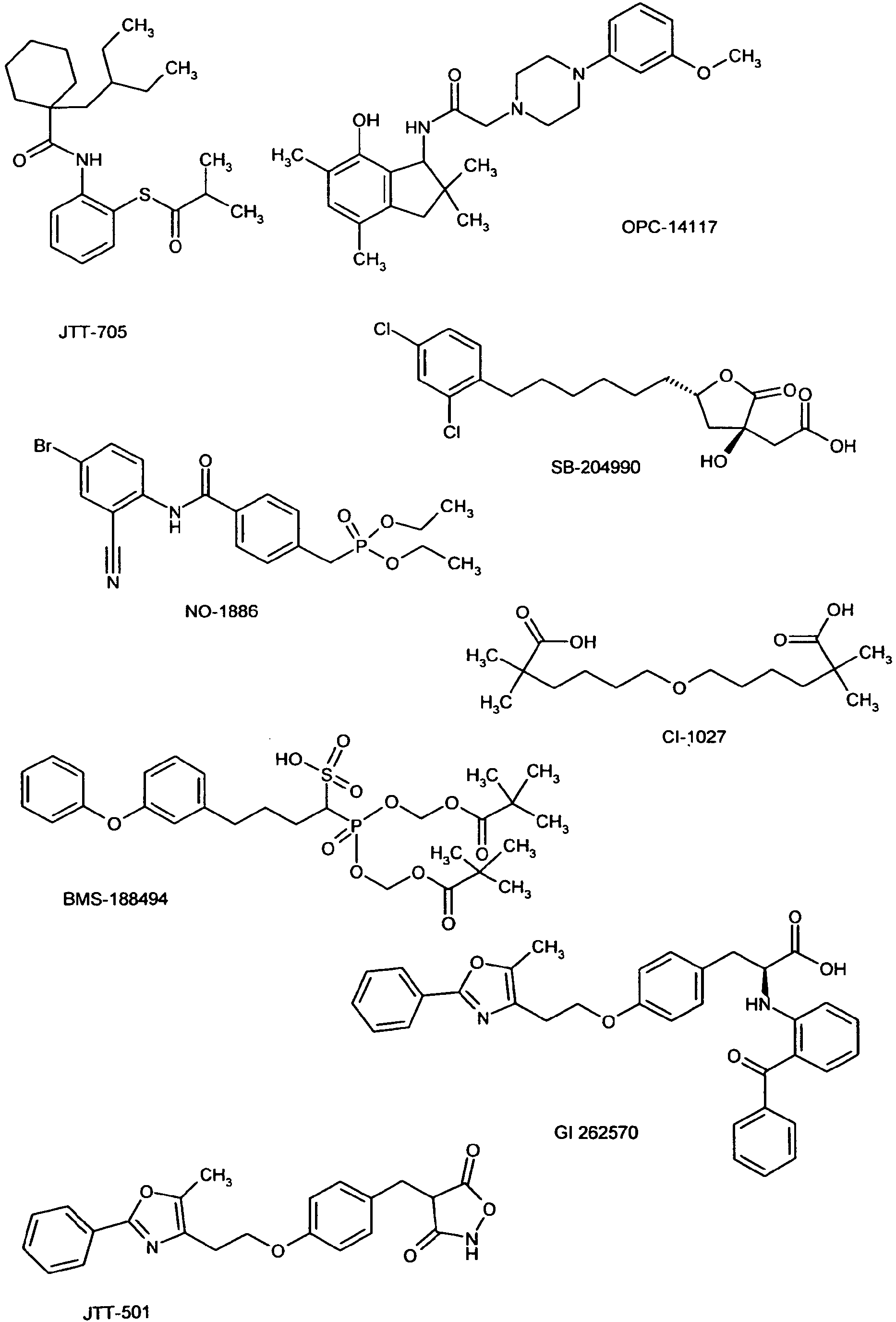 Die nachfolgend aufgeführten Beispiele dienen zur Erläuterung der Erfindung, ohne diese jedoch einzuschränken.

| Beispiel Nr. | Formel | Massenspektro-metrische Daten |
|---|---|---|
| 1 | 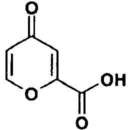 | MS(ESI⁺) 141,04 |
| 2 | 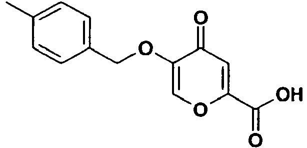 | MS(ESI⁺) 261,12 |
| 3 | 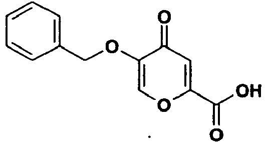 | MS(ESI⁻) 245,54 |
| 4 | 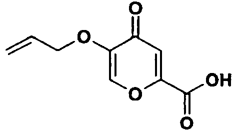 | MS(ESI⁺) 197,11 |
| 5 | 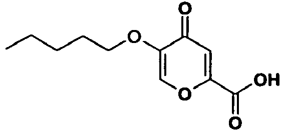 | MS(ESI⁺) 227,15 |
| 6 | 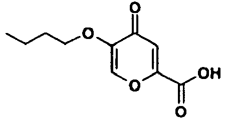 | MS(ESI⁺) 213,11 |
| 7 | 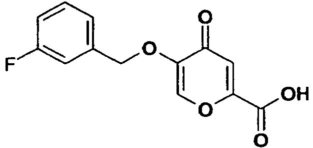 | MS(ESI⁺) 265,22 |
| 8 | 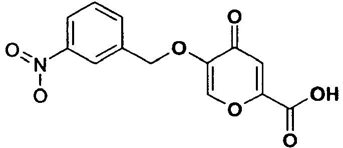 | MS(ESI⁺) 292,13 |
| 9 | 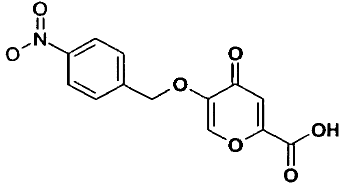 | |
| 10 | 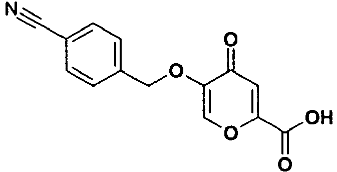 | MS(ESI⁻) 270,41 |
| 11 | 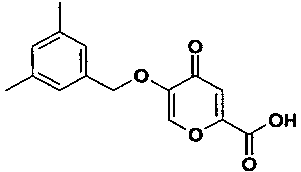 | MS(ESI⁺) 275,14 |
| 12 | 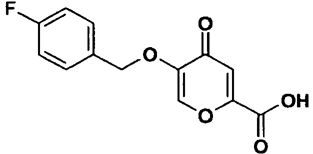 | MS(ESI⁻) 263,36 |
| 13 | 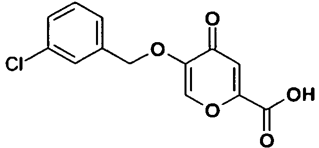 | MS(ESI⁻) 279,32 |
| 14 | 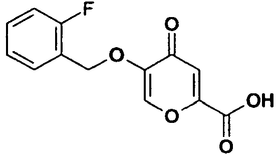 | MS(ESI⁻) 263,29 |
| 15 | 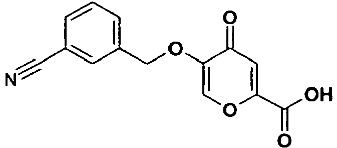 | MS(ESI⁻) 270,28 |
| 16 | 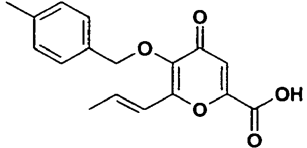 | MS(ESI⁺) 345,10 (M+HCOO-) |
| 17 | 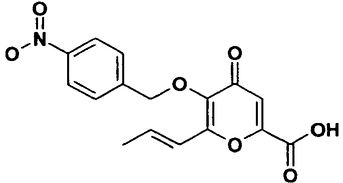 | |
| 18 | 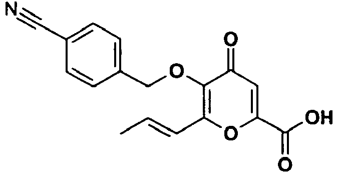 | |
| 19 | 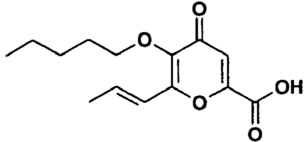 | MS(ESI⁺) 267,12 |
| 20 | 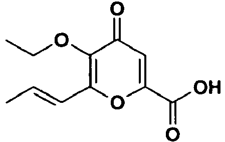 | |
| 21 | 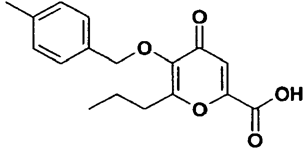 | MS(ESI⁺) 303,13 |
| 22 | 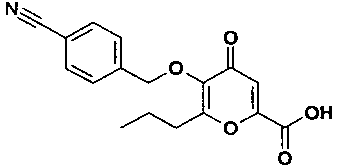 | MS(ESI⁺) 314,10 |
| 23 | 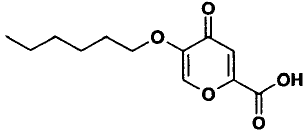 | MS(ESI⁺) 241,08 |
| 24 | 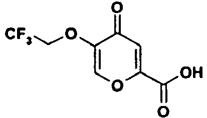 | MS(ESI⁺) 239,06 |
| 25 | 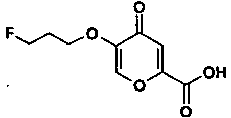 | MS(ESI⁺) 217,05 |
| 26 | 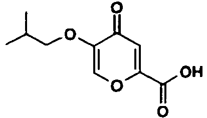 | MS(ESI⁺) 213,03 |
| 27 | 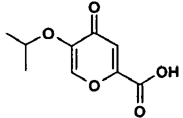 | MS(ESI⁺) 199,10 |
| 28 | 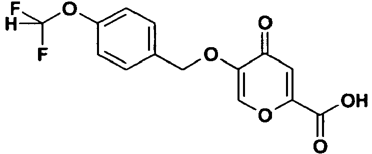 | MS(ESI⁺) 313,07 |
| 29 | 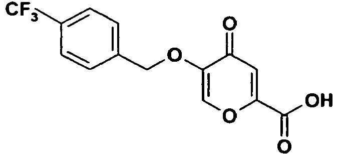 | MS(ESI⁺) 315,22 |
| 30 | 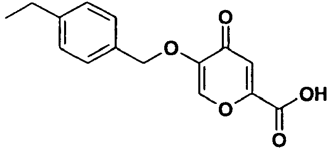 | MS(ESI⁺) 275,19 |
| 31 | 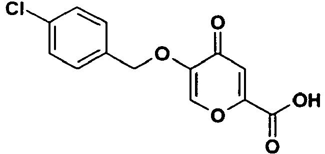 | MS(ESI⁺) 281,05 |
| 32 | 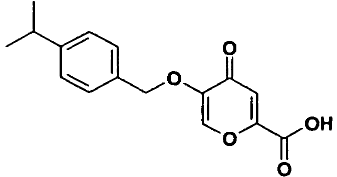 | MS(ESI⁺) 289,13 |
| 33 | 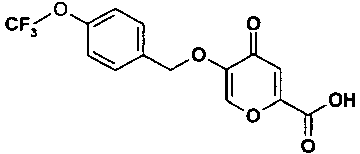 | MS(ESI⁺) 331,02 |
| 34 | 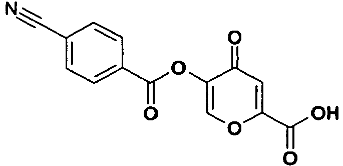 | MS(ESI⁻) 569,04 (2M-H⁺) |
| 35 | 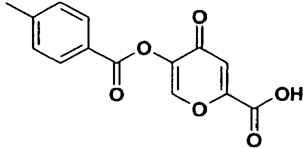 | MS(ESI⁺) 275,10 |
| 36 | 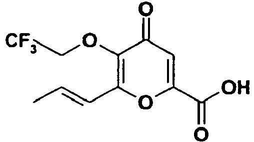 | MS(ESI⁺) 279,05 |
| 37 | 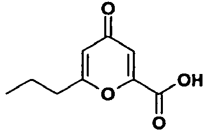 | MS(ESI⁺) 183,11 |
| 38 | 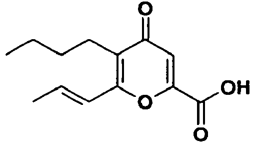 | MS(ESI⁺) 237,16 |
| 39 | 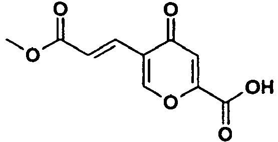 | MS(ESI⁺) 266,10 (M+CH₃CN+H⁺) |
| 40 | 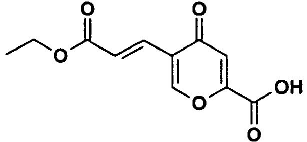 | MS(ESI⁺) 239,09 |
| 41 | 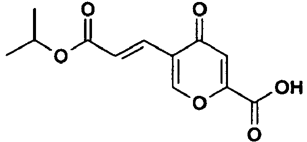 | MS(ESI⁻) 251,17 |
| 42 | 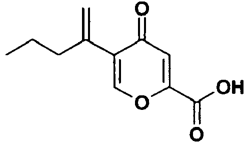 | MS(ESI⁺) 209,09 |
| 43 | 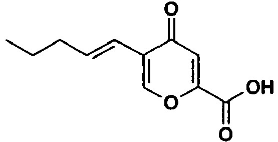 | MS(ESI⁺) 209,09 |
| 44 | 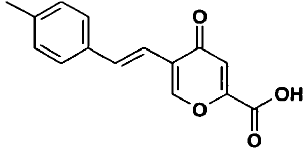 | MS(ESI⁺) 257,16 |
| 45 | 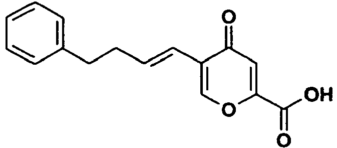 | MS(ESI⁺) 271,12 |
| 46 | 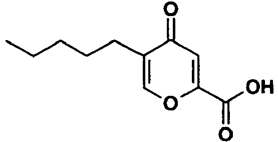 | MS(ESI⁺) 252,12 (M+CH₃CN+H⁺) |
| 47 | 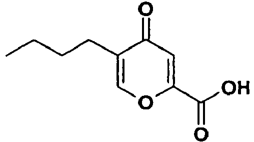 | MS(ESI⁺) 197,13 |
| 48 | 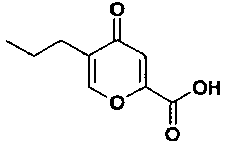 | MS(ESI⁺) 183,09 |
| 49 | 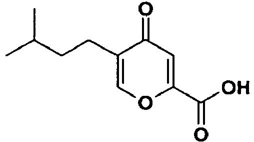 | MS(ESI⁺) 252,20 (M+CH₃CN+H⁺) |
| 50 | 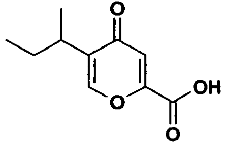 | MS(ESI⁺) 197,10 |
| 51 | 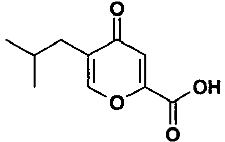 | MS(ESI⁺) 238,11 (M+CH₃CN+H⁺) |
| 52 | 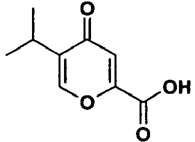 | MS(ESI⁺⁻) 181,15 |

Die Verbindungen der Formel I eignen sich zur Behandlung des Metabolischen Syndroms (siehe Datamonitor 11/2002, Kapitel 2, Seiten 19- 32), zur Prädiabetes Behandlung und zur Prophylaxe von Diabetes Typ 2. Sie einen sich insbesonders zur Behandlung der Diabetischen Dislipidämie. Die diabetische Dyslipidämie manifestiert sich in einer Erhöhung der Plasmatriglyzeride, einer Verminderung des HDL-Cholesterins sowie oft in erhöhten LDL-Spiegeln. Durch das gehäufte Auftreten kleiner, dichter LDL-Cholesterinpartikel mit hoher atherogener Potenz ist die Diabetische Dyslipidämie ein starker kardiovaskulärer Risikofaktor.

Weiter eignen sich diese Verbindungen zur Behandlung und/oder Prävention von
1.
   - Störungen des Fettsäurestoffwechsels und Glucoseverwertungsstörungen
   - Störungen, bei denen Insulin Resistenz eine Rolle spielt
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
   - Dyslipidämien und deren Folgen, wie z.B. Atherosklerose, koronare Herzkrankheit, zerebrovaskuläre Erkrankungen etc, insbesondere solche (aber nicht beschränkt auf)
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt , hypertensive Herzerkrankung oder Kardiomyopathie
2. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und hamableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

Die Verbindungen der Formel I können beispielsweise in folgenden Zubereitungen formuliert werden:

### Beispiel A

Gelatineweichkapseln, enthaltend 100 mg Wirkstoff pro Kapsel:

| | pro Kapsel |
|---|---|
| Wirkstoff | 100 mg |
| aus Kokosfett fraktioniertes | |
| Triclycerid-Gemisch | 400 mg |
| Kapselinhalt | 500 mg |

### Beispiel B

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

### Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

### Beispiel E

Dragees, enthaltend 50 mg Wirkstoff pro Dragees:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

### Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

Die Wirksamkeit der Verbindungen der Formel I wurde wie folgt getestet:

### Biologisches Prüfmodell:

Die Prüfung der Wirkung erfolgte

Am Ganztier (Maus, Ratte, Hamster oder Hund) wird nach einer Fastenperiode (z.B. von ca. 16 Stunden, die Substanz appliziert (z.B. p.o., iv., i.p., s.c. ) und mit oder ohne zusätzliche Stimulation der endogenen Lipolyse (z.B. durch eine Bolusinjektion von 2 mg/kg i.p. Isoprenaline) die Wirkung der Testsubstanz auf die Lipolyse anhand der freigesetzten freien Fettsäuren (FFA), Glycerol und Triglyceride bestimmt, indem z.B. 15 min, 30, 60, 120 usw. Minuten nach p.o. Applikation eine Blutprobe (z.B. durch retroorbitale Blutentnahme) gewonnen wird und nach Standard klinisch-chemischen Methoden (z.B. L.Thomas: Labor und Diagnose, 2. Auflage, Medizinische Verlagsgesellschaft, Marburg/L. 1984; ISBN 3-921320-10-9)) analysiert wird. Die Hemmung der Lipolyse durch die Inhibitoren wird im Vergleich zur Lipolyserate an entsprechend behandelten Kontrolltieren ausgewertet.

Beispiel 1 wurde in der Dosis von 3 mg/kg po verabreicht, wodurch die Lipolyse deutlich abgesenkt wurde, wie an der Reduktion von freien Fettsäuren (FFA), Glycerol und Triglyceriden gezeigt werden konnte.

| FFA [mmol/L] | | |
|---|---|---|
| Zeitpunkt | Kontrolle n=14 | 3mg/kg n=7 |
| 0 | 0,54 | 0,55 |
| 15 min | 0,59 | 0,22 |
| 30 min | 0,74 | 0,14 |
| 60 min | 0,61 | 0,16 |
| 120 min | 0,75 | 0,28 |

| Glycerin [µmol/L] | | |
|---|---|---|
| Zeitpunkt | Kontrolle n=14 | 3mg/kg n=7 |
| 0 | 109 | 117 |
| 15 min | 94 | 55 |
| 30 min | 129 | 43 |
| 60 min | 99 | 43 |
| 120 min | 126 | 74 |

| Triglyceride [mmol/L] | | |
|---|---|---|
| Zeitpunkt | Kontrolle n=16 | 3mg/kg n=7 |
| 0 | 0,49 | 0,53 |
| 15 min | 0,46 | 0,48 |
| 30 min | 0,41 | 0,28 |
| 60 min | 0,39 | 0,21 |
| 120 min | 0,46 | 0,25 |

Beispiel 47 wurde in der Dosis von 1 mg/kg po verabreicht, wodurch die Lipolyse deutlich abgesenkt wurde, wie an der Reduktion von freien Fettsäuren (FFA), Glycerol und Triglyceriden gezeigt werden konnte.

| FFA [mmol/L] | | |
|---|---|---|
| Zeitpunkt | Kontrolle n=6 | 1mg/kg n=6 |
| 0 | 0,80 | 0,84 |
| 30 min | 0,50 | 0,25 |
| 60 min | 0,56 | 0,37 |
| 120 min | 0,59 | 0,41 |

| Glycerin [µmol/L] | | |
|---|---|---|
| Zeitpunkt | Kontrolle n=6 | 1mg/kg n=6 |
| 0 | 160 | 162 |
| 30 min | 103 | 41 |
| 60 min | 104 | 55 |
| 120 min | 106 | 70 |

| Triglyceride [mmol/L] | | |
|---|---|---|
| Zeitpunkt | Kontrolle n=6 | 1mg/kg n=6 |
| 0 | 0,66 | 0,84 |
| 30 min | 0,87 | 0,25 |
| 60 min | 0,67 | 0,37 |
| 120 min | 0,56 | 0,41 |

Beispiel 48 wurde in der Dosis von 10 mg/kg po verabreicht, wodurch die Lipolyse deutlich abgesenkt wurde, wie an der Reduktion von freien Fettsäuren (FFA), Glycerol und Triglyceriden gezeigt werden konnte.

| FFA [mmol/L] | | |
|---|---|---|
| Zeitpunkt | Kontrolle n=16 | 10mg/kg n=6 |
| 0 | 0,53 | 0,51 |
| 30 min | 0,49 | 0,19 |
| 60 min | 0,48 | 0,19 |
| 120 min | 0,49 | 0,16 |

| Glycerin [µmol/L] | | |
|---|---|---|
| Zeitpunkt | Kontrolle n=16 | 10mg/kg n=6 |
| 0 | 115 | 100 |
| 30 min | 97 | 35 |
| 60 min | 89 | 26 |
| 120 min | 91 | 24 |

| Triglyceride [mmol/L] | | |
|---|---|---|
| Zeitpunkt | Kontrolle n=16 | 10mg/kg n=6 |
| 0 | 0,35 | 0,41 |
| 30 min | 0,45 | 0,36 |
| 60 min | 0,40 | 0,25 |
| 120 min | 0,35 | 0,27 |

Beispiel 50 wurde in der Dosis von 3 mg/kg po verabreicht, wodurch die Lipolyse deutlich abgesenkt wurde, wie an der Reduktion von freien Fettsäuren (FFA), Glycerol und Triglyceriden gezeigt werden konnte.

| FFA [mmol/L] | | |
|---|---|---|
| Zeitpunkt | Kontrolle n=12 | 3mg/kg n=6 |
| 0 | 0,59 | 0,64 |
| 15 min | 0,62 | 0,34 |
| 30 min | 0,59 | 0,43 |
| 60 min | 0,56 | 0,50 |
| 120 min | 0,56 | 0,52 |

| Glycerin [µmol/L] | | |
|---|---|---|
| Zeitpunkt | Kontrolle n=12 | 3mg/kg n=6 |
| 0 | 130 | 127 |
| 15 min | 121 | 67 |
| 30 min | 121 | 82 |
| 60 min | 120 | 86 |
| 120 min | 107 | 101 |

| Triglyceride [mmol/L] | | |
|---|---|---|
| Zeitpunkt | Kontrolle n=12 | 3mg/kg n=6 |
| 0 | 0,44 | 0,47 |
| 15 min | 0,48 | 0,39 |
| 30 min | 0,56 | 0,36 |
| 60 min | 0,55 | 0,58 |
| 120 min | 0,46 | 0,71 |

"n" steht für die Anzahl der Tiere. Es wurden Wistar Ratten getestet.

Agonisten des G-Protein gekoppelten Rezeptors HM74a (GPR109a) führen zu einer Abnahme von cyclischem Adenosin Monophosphat (cAMP) und zur Inhibition der Lipolyse in Adipocyten (S. Offermanns, Trends in Pharm Sciences 2006, 27, 384-390.).

Verbindungen, die zu einer Aktivierung des Rezeptors HM74a führen, sind für eine Behandlung von Krankheiten geeignet, die durch HM74a Agonisten moduliert werden.

Die Wirksamkeit der Verbindungen der Formel I wurde in folgenden Assays getestet:

In vitro funktionale Assays mit rekombinanten Zellen

Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die sowohl den GPCR HM74A (Niacin-Receptor), als auch das hybride G-protein Gα6qi4myr (siehe z.B. Patentanmeldung DE10033353) exprimieren.

Für die Untersuchungen wurden Zellen in 96-well-Mikrotiterplatten (60000 Zellen/well) ausgesät und übernacht wachsengelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0%: keine Testsubstanz addiert; 100%: 1 µM Referenzagonist Niacin addiert), zur Berechnung von Dosis/Wirkungskurven verwendet und EC50-Werte bestimmt.

**Tabelle 2: Biologische Aktivität**

| Beispiel Nr. | EC50 (HM74a) µM |
|---|---|
| 1 | 0.09 |
| 2 | 4.12 |
| 3 | 28.80 |
| 4 | 29.94 |
| 5 | 1.20 |
| 6 | 52.87 |
| 7 | 62.84 |
| 8 | 36.85 |
| 9 | 43.37 |
| 10 | 14.74 |
| 11 | 31.23 |
| 12 | 49.49 |
| 13 | 19.50 |
| 14 | 12.12 |
| 15 | 67.13 |
| 16 | 0.10 |
| 17 | 0.24 |
| 18 | 0.36 |
| 19 | 0.39 |
| 20 | 0.73 |
| 21 | 4.40 |
| 22 | 45.03 |
| 23 | 34.25 |
| 24 | 1.00 |
| 25 | 0.76 |
| 26 | 45.27 |
| 27 | 4.61 |
| 28 | 1.81 |
| 29 | 45.07 |
| 30 | 3.24 |
| 31 | 7.43 |
| 32 | 1.48 |
| 33 | 22.36 |
| 34 | 0.31 |
| 35 | 1.19 |
| 36 | 0.07 |
| 37 | 66.66 |
| 38 | 0.14 |
| 39 | 1.19 |
| 40 | 1.19 |
| 41 | 6.87 |
| 42 | 6.60 |
| 43 | 1.03 |
| 44 | 4.83 |
| 45 | 10.85 |
| 46 | 0.01 |
| 47 | 0.01 |
| 48 | 0.05 |
| 49 | 0.21 |
| 50 | 0.16 |
| 51 | 0.22 |
| 52 | 2.69 |

### Synthese:

Die Verbindung des Beispiels 1 wurde von einem Chemikalienanbieter (ABCR GmbH KG, Karlsruhe, Germany) gekauft.

### Synthese von 5-(4-Methyl-benzyloxy)-4-oxo-4H-pyran-2-carbonsäure (Beispiel 2):

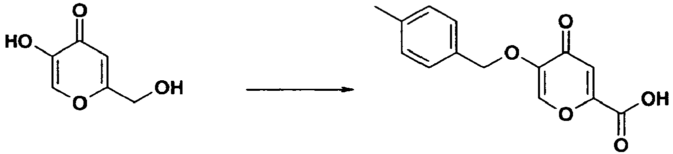

500 mg 5-Hydroxy-2-hydroxymethyl-pyran-4-on (Kojisäure) wurden in 20 mL DMF gelöst, mit 0,47 mL 4-Methylbenzylbromid und 0,97 g Kaliumcarbonat versetzt und 17h bei Raumtemperatur gerührt. Die Reaktionslösung wurde mit 50 mL Dichlormethan versetzt und mit 70 mL einer gesättigten Lösung von Ammoniumchlorid in Wasser extrahiert. Die organische Phase wurde abgetrennt und am Rotationsverdampfer eingeengt. Das Rohprodukt wurde in 100 mL Acetonitril gelöst und mit 2,4 ml einer Lösung aus 25g CrO₃ und 25 mL H₂SO₄ in 100 mL Wasser versetzt. Nach 1,5h wurde die Reaktionslösung mit 100ml Methyl-tert.-Buthylether versetzt. Anschließend wurde die Reaktionslösung 5 mal mit je 100 mL einer 1 molaren HCl-Lösung extrahiert. Die organische Phase wurde über Magnesiumsulfat getrocknet und am Rotationsverdampfer eingeengt. Anschließend wurde das Rohprodukt an einer HPLC (Agilent-Prep.-C 18, Laufmittel MeCN / H₂O / TFA) gereinigt. Man erhält 63 mg (0,263 mmol) 5-(4-Methyl-benzyloxy)-4-oxo-4H-pyran-2-carbonsäure als farbloses Wachs.
LCMS: m = 261,12 (M+H)⁺

Die Verbindungen der Beispiele 3-15 und 23-35 wurden analog dieser allgemeinen Arbeitsvorschrift ausgehend von Kojisäure synthetisiert.

### Synthese von 5-(4-Methyl-benzyloxy)-4-oxo-6-propenyl-4H-pyran-2-carbonsäure (Beispiel 16):

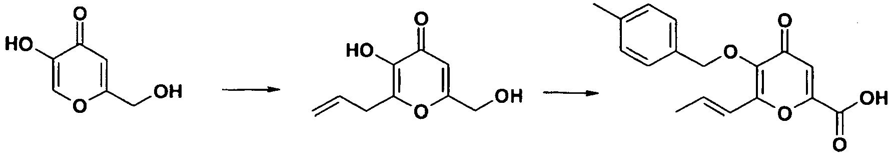

5,00 g 5-Hydroxy-2-hydroxymethyl-pyran-4-on (Kojisäure) wurden in 50 mL DMF gelöst, mit 3,22 mL Allylbromid und 9,73 g Kaliumcarbonat versetzt und 4 h bei Raumtemperatur gerührt. Die Reaktionslösung wurde am Rotationsverdampfer auf 20 mL reduziert und mit 250 mL Essigsäureethylester und 30 mL Wasser versetzt. Die organische Phase wurde abgetrennt, über Magnesiumsulfat getrocknet und am Rotationsverdampfer eingeengt. Das Rohprodukt wurde in 40 mL Toluol aufgenommen und 16 h unter Rückfluß erhitzt. Nach Abkühlen fiel ein farbloser Niederschlag aus, der abfiltriert wurde und mit 20 mL kaltem Toluol gewaschen wurde. Man erhielt 5,13 g 2-Allyl-3-hydroxy-6-hydroxymethyl-pyran-4-on als farblosen Feststoff. LCMS: m = 183 (M+H)⁺. 2-Allyl-3-hydroxy-6-hydroxymethyl-pyran-4-on wurde analog der Synthese der Verbindung von Beispiel 2 zu 5-(4-Methyl-benzyloxy)-4-oxo-6-propenyl-4H-pyran-2-carbonsäure umgesetzt. LCMS: m = 345,10 (M+HCOO⁻).

Die Verbindungen der Beispiele 17-20 und 36 wurden analog dieser allgemeinen Arbeitsvorschrift ausgehend von Kojisäure synthetisiert.

### Synthese von 5-(4-Methyl-benzyloxy)-4-oxo-6-propyl-4H-pyran-2-carbonsäure (Beispiel 21):

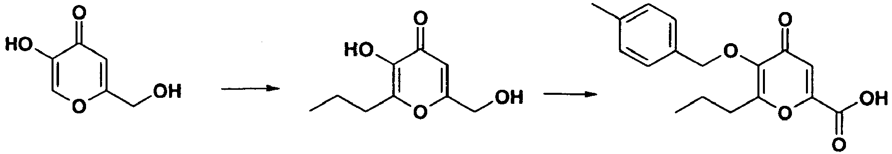

5,00 g 5-Hydroxy-2-hydroxymethyl-pyran-4-on (Kojisäure) wurden in 50 mL DMF gelöst, mit 3,22 mL Allylbromid und 9,73 g Kaliumcarbonat versetzt und 4 h bei Raumtemperatur gerührt. Die Reaktionslösung wurde am Rotationsverdampfer auf 20 mL reduziert und mit 250 mL Essigsäureethylester und 30 mL Wasser versetzt. Die organische Phase wurde abgetrennt, über Magnesiumsulfat getrocknet und am Rotationsverdampfer eingeengt. Das Rohprodukt wurde in 40 mL Toluol aufgenommen und 16 h unter Rückfluß erhitzt. Nach Abkühlen fiel ein farbloser Niederschlag aus, der abfiltriert wurde und mit 20 mL kaltem Toluol gewaschen wurde. Der erhaltene Feststoff wurde in 25 mL Methanol gelöst, mit 350 mg Palladium auf Kohle (10%) versetzt und im Autoklaven unter Wasserstoffatmosphäre (1,5 bar) für 24 h gerührt. Palladium auf Kohle wurde abfiltriert, und die Reaktionslösung wurde am Rotationsverdampfer eingeengt. Man erhielt 5,04 g 2-Propyl-3-hydroxy-6-hydroxymethyl-pyran-4-on als farblosen Feststoff. LCMS: m = 185 (M+H)⁺. 2-Propyl-3-hydroxy-6-hydroxymethyl-pyran-4-on wurde analog der Synthese der Verbindung von Beispiel 2 zu 5-(4-Methyl-benzyloxy)-4-oxo-6-propyl-4H-pyran-2-carbonsäure umgesetzt. LCMS: m = 303,13 (M+H)⁺.

Die Verbindung des Beispiels 22 wurden analog dieser allgemeinen Arbeitsvorschrift ausgehend von Kojisäure synthetisiert.

### Synthese von 5-Propyl-4-oxo-4H-pyran-2-carbonsäure (Beispiel 48):

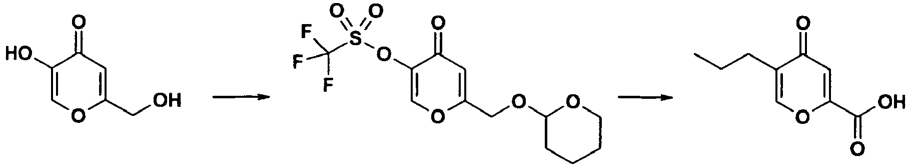

16,7 g 5-Hydroxy-2-hydroxymethyl-pyran-4-on (Kojisäure) wurden in 200 mL Dichlormethan suspendiert und mit 15,8 mL 3,4-Dihydro-2H-pyran, und 160 mg Toluolsulfonsäure versetzt. Das Reaktionsgemisch wurde 4h bei Raumtemperatur gerührt und 2 mal mit jeweils 100 mL 2-molarer wässriger NaOH-Lösung extrahiert. Die wässrige Lösung wurde mit gesättigter, wässriger NaH₂PO₄-Lösung auf pH=7 eingestellt, und mit 200 mL Dichlormethan extrahiert. Die organische Phase wurde über Magnesiumsulfat getrocknet und am Rotationsverdampfer eingeengt. Das erhaltene Produkt wurde in in 200 mL Dichlormethan gelöst, auf 0°C gekühlt, mit 14 mL Pyridin und 17,6 mL Trifluormethansulfonsäureanhydrid versetzt. Nach 16 h wurde das Reaktionsgemisch mit 200 mL gesättigter, wässriger NH₄Cl-Lösung gewaschen, über Magnesiumsulfat getrocknet und Flashchromatographisch (Laufmittel: Essigsäureethylester/Heptan) gereinigt. Man erhielt 20,1 g Trifluormethanesulfonsäure-4-oxo-6-(tetrahydropyran-2-yloxymethyl)-4H-pyran-3-yl-ester als farblosen Feststoff.

1,5 g Trifluormethanesulfonsäure-4-oxo-6-(tetrahydropyran-2-yloxymethyl)-4H-pyran-3-yl-ester wurden in 40 mL Toluol gelöst und mit 47 mg Palladium(II)acetat, 260 mg BINAP, 2,73 g Caesiumcarbonat und 442 mg n-Propylboronsäure versetzt. Das Reaktionsgemisch wurde 6h bei 100°C erhitzt. Die Feststoffe wurden abfiltriert, und das Reaktionsgemisch wurde am Rotationsverdampfer eingeengt. Das Rohprodukt wurde flashchromatographisch gereinigt (Laufmittel: Essigsäureethylester/Heptan) und anschließend in 20 mL Acetonitril gelöst, und mit 2,7 ml einer Lösung aus 25g CrO₃ und 25 mL H₂SO₄ in 100 mL Wasser versetzt. Nach 1 h wurde die Reaktionslösung mit 100 mL Methyl-tert.-Buthylether versetzt. Anschließend wurde die Reaktionslösung 2 mal mit je 30 mL einer 1 molaren HCl-Lösung extrahiert. Die organische Phase wurde über Magnesiumsulfat getrocknet und am Rotationsverdampfer eingeengt. Anschließend wurde das Rohprodukt an einer HPLC (Agilent-Prep.-C18, Laufmittel MeCN /H₂O̅ / TFA) gereinigt. Man erhält 194 mg (1,07 mmol) 5-Propyl-4-oxo-4H-pyran-2-carbonsäure als farblosen Feststoff. LCMS: m = 183,09 (M+H)⁺.

Die Verbindungen der Beispiele 42-43 und der Beispiele 46-47 wurden analog dieser allgemeinen Arbeitsvorschrift ausgehend von Kojisäure synthetisiert.

Die Verbindung 38 wurde analog dieser allgemeinen Arbeitsvorschrift ausgehend von 2-Allyl-3-hydroxy-6-hydroxymethyl-pyran-4-on synthetisiert.

### Synthese von 6-Propyl-4-oxo-4H-pyran-2-carbonsäure (Beispiel 37):

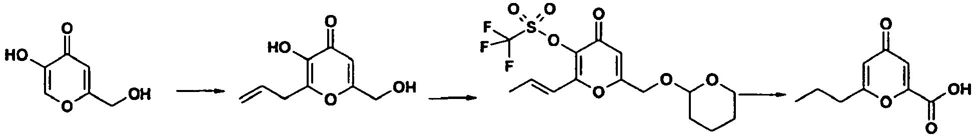

25,0 g 2-Allyl-3-hydroxy-6-hydroxymethyl-pyran-4-on wurden in 500 mL Dichlormethan gelöst, mit 18,4 mL 3,4-Dihydro-2H-pyran, und 182 mg Toluolsulfonsäure versetzt. Das Reaktionsgemisch wurde 2h bei Raumtemperatur gerührt und 2 mal mit jeweils 100 mL 2-molarer wässriger NaOH-Lösung extrahiert. Die wässrige Lösung wurde mit gesättigter, wässriger NaH₂PO₄-Lösung auf pH=7 eingestellt, und mit 400 mL Dichlormethan extrahiert. Die organische Phase wurde über Magnesiumsulfat getrocknet und am Rotationsverdampfer eingeengt. Das erhaltene Produkt wurde in in 222 mL Dichlormethan suspendiert, auf 0°C gekühlt, mit 12,8 mL Pyridin und 22,2 mL Trifluormethansulfonsäureanhydrid versetzt. Nach 2 h wurde das Reaktionsgemisch mit 200 mL gesättigter, wässriger NH₄Cl-Lösung gewaschen, über Magnesiumsulfat getrocknet und Flashchromatographisch (Laufmittel: Essigsäureethylester/Heptan) gereinigt. Man erhielt 15,8 g Trifluormethanesulfonsäure-4-oxo-2-((E)-propenyl)-6-(tetrahydropyran-2-yloxymethyl)-4H-pyran-3-yl-ester als farblosen Feststoff.

7,00 g Trifluormethanesulfonsäure-4-oxo-2-((E)-propenyl)-6-(tetrahydropyran-2-yloxy-methyl)-4H-pyran-3-yl-ester wurden in 40,8 mL DMF gelöst und mit 75,8 mg Palladium(II)acetat, 154 mg Tri-(o-tolyl)-phosphin, 7,07 mL Triethylamin und 0,64 mL Ameisensäure versetzt. Das Reaktionsgemisch wurde 1h bei 60°C erhitzt.

Die Feststoffe wurden abfiltriert, und das Reaktionsgemisch wurde am Rotationsverdampfer eingeengt. Das Rohprodukt wurde flashchromatographisch gereinigt (Laufmittel: Essigsäureethylester/Heptan) und anschließend in 46,7 mL Acetonitril gelöst, und mit 2,0 ml einer Lösung aus 25g CrO₃ und 25 mL H₂SO₄ in 100 mL Wasser versetzt. Nach 1 h wurde die Reaktionslösung mit 100 mL Methyl-tert.-Buthylether versetzt. Anschließend wurde die Reaktionslösung 3 mal mit je 30 mL einer 1-molaren HCl-Lösung extrahiert. Die organische Phase wurde über Magnesiumsulfat getrocknet und am Rotationsverdampfer eingeengt. Anschließend wurde das Rohprodukt an einer HPLC (Agilent-Prep.-C18, Laufmittel MeCN /H₂O / TFA) gereinigt. Man erhält 7 mg 6-Propyl-4-oxo-4H-pyran-2-carbonsäure als farblosen Feststoff. LCMS: m = 183,11 (M+H)⁺.

### Synthese von 5-((E)-2-Ethoxycarbonyl-vinyl)-4-oxo-4H-pyran-2-carbonsäure (Beispiel 40):

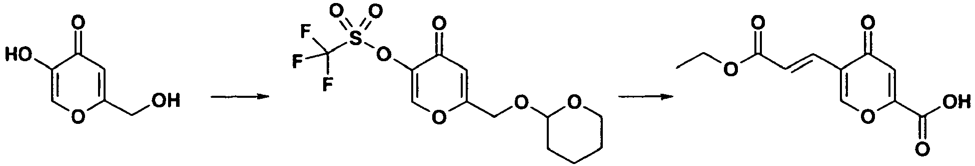

3,00 g Trifluormethanesulfonsäure-4-oxo-6-(tetrahydropyran-2-yloxymethyl)-4H-pyran-3-yl-ester werden in 200 mL THF gelöst, mit 1,09 g Ethylacrylat, mit 37,6 mg Palladium(II)acetat, mit 3,46 mL Triethylamin, und mit 102 mg Tri-(o-tolyl)-phosphin vesetzt. Die Lösung wurde 6h bei 80°C gerührt. Gebildete Niederschläge wurden durch Filtration entfernt. Die organische Phase wurde am Rotationsverdampfer eingeengt, und Flashchromatographisch (Laufmittel: Essigsäureethyleste/Heptan) gereinigt. Man erhält 507 mg 5-((E)-2-Ethoxycarbonyl-vinyl)-2-(tetrahydropyran-2-yloxymethyl)-pyran-4-on, die in 15 mL Acetonitril gelöst wurden, und mit 1,80 ml einer Lösung aus 25g CrO₃ und 25 mL H₂SO₄ in 100 mL Wasser versetzt wurden. Nach 1 h wurde die Reaktionslösung mit 60 mL Methyl-tert.-buthylether versetzt. Anschließend wurde die Reaktionslösung 2 mal mit je 100 mL einer 1-molaren HCl-Lösung extrahiert. Die organische Phase wurde über Magnesiumsulfat getrocknet und am Rotationsverdampfer eingeengt. Anschließend wurde das Rohprodukt an einer HPLC (Agilent-Prep.-C 18, Laufmittel MeCN / H₂O / TFA) gereinigt. Man erhält 110 mg von 5-((E)-2-Ethoxycarbonyl-vinyl)-4-oxo-4H-pyran-2-carbonsäure als farblosen Feststoff. LCMS: m = 239,09 (M+H)⁺.

Die Verbindungen der Beispiele 39, 41, 44 und 45 wurden analog dieser allgemeinen Arbeitsvorschrift ausgehend von Kojisäure synthetisiert.

### Synthese von 5-(3-Methyl-butyl)-4-oxo-4H-pyran-2-carbonsäure (Beispiel 49):

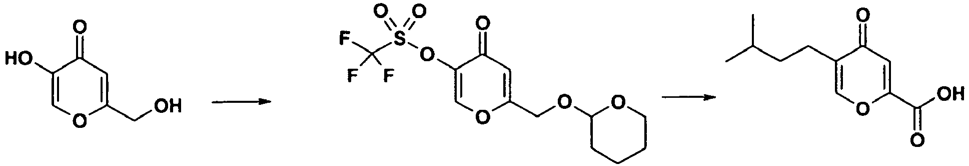

2,28 g Trifluormethanesulfonsäure-4-oxo-6-(tetrahydropyran-2-yloxymethyl)-4H-pyran-3-yl-ester werden in 35 mL THF gelöst, bei 0°C mit 25,46 mL einer 0,5 molaren Lösung von 3-Methylbutylzinkbromid in THF, mit 71,44 mg Palladium(II)acetat, mit 4,15 g Caesiumcarbonat, und mit 396 mg BINAP vesetzt. Die Lösung wurde 1h bei RT gerührt und anschließend 5h unter Rückfluß erhitzt. Gebildete Niederschläge wurden durch Filtration entfernt. Die organische Phase wurde am Rotationsverdampfer eingeengt. Der Rückstand wurde in 200 mL Essigsäureethylester aufgenommen, mit 100 mL gewaschen, über Magnesiumsulfat getrocknet, am Rotationsverdampfer eingeengt, und Flashchromatographisch (Laufmittel: Essigsäureethyleste/Heptan) gereinigt. Man erhält 210 mg 5-(3-Methyl-butyl)-2-(tetrahydropyran-2-yloxymethyl)-pyran-4-on, die in 20 mL Acetonitril gelöst wurden, und mit 0,75 ml einer Lösung aus 25g CrO₃ und 25 mL H₂SO₄ in 100 mL Wasser versetzt wurden. Nach 1,5 h wurde die Reaktionslösung mit 100 mL Methyl-tert.-Buthylether versetzt. Anschließend wurde die Reaktionslösung 4 mal mit je 50 mL einer 1-molaren HCl-Lösung extrahiert. Die organische Phase wurde über Magnesiumsulfat getrocknet und am Rotationsverdampfer eingeengt. Anschließend wurde das Rohprodukt an einer HPLC (Agilent-Prep.-C18, Laufmittel MeCN / H₂O / TFA) gereinigt. Man erhält 35 mg (0,166 mmol) 5-(3-Methyl-butyl)-4-oxo-4H-pyran-2-carbonsäure als farbloses Wachs. LCMS: m = 252,20 (M+H+MeCN)⁺.

Die Verbindungen der Beispiele 49-52 wurden analog dieser allgemeinen Arbeitsvorschrift ausgehend von Kojisäure synthetisiert.

## Patentansprüche

1. Verwendung der Verbindungen der Formel I, 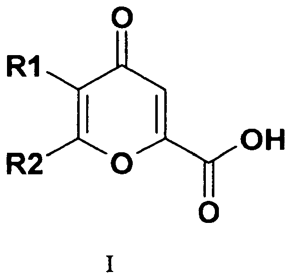 worin bedeuten
R1 H, F, Cl, Br, I, OH, CF₃, NO₂, CN, OCF₃, NH₂, NH(C₁-C₆)-Alkyl, N((C₁-C₆)-Alkyl)₂, SF₅, SO₂-CH₃, COOH, COO-(C₁-C₆)-Alkyl, CONH₂, (C₁-C₂₀)-Alkyl, (C₃-C₂₀)-Cycloalkyl, (C₂-C₂₀)-Alkenyl, (C₂-C₂₀)-Alkinyl, Aryl, Heterozyklus, wobei in den (C₁-C₂₀)-Alkyl, (C₂-C₂₀)-Alkenylresten ein oder mehrere einzelne -CH₂- oder-CH- Gruppen durch -O- ersetzt sein können und wobei die Alkyl-, Cycloalkyl-, Alkenyl-, Alkinyl-, Aryl- und Heterozyklylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]_{2,} Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder
Heterozyklische Rest bis zu zweifach mit F, Cl, Br, OH, CF₃, SF₅, NO₂, CN, OCF₃, O-(C,-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl),N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-_{A}lkyl)-Aryl, N[(C₁-C₆)-Alkyl]-CO-N((C₁-C₆)-_{A}lkyl)-Heterozyklyl, N[(C₁-C₆)-Alkyl]-CO-N(Aryl)_{2,} N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C ₁-C₆)-Alkyl)₂, N(Heterozyklyl)-CO-N((C₁-C₆)-Alkyl)₂, N(Aryt)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C)-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
R2 H, F, Cl, Br, I, OH, CF₃, NO₂, CN, OCF₃, NH₂, NH(C₁-C₆)-Alkyl, N((C₁-C₆)-Alkyl)₂, SF₅, SO₂-CH₃, COOH, COO-(C₁-C₆)-Alkyl, CONH₂, (C₁-C₂₀)-Alkyl, (C₃-C₂₀)-Cycloalkyl, (C₂-C₂₀)-Alkenyl, (C₂-C₂₀)-Alkinyl, Aryl, Heterozyklus, wobei in den (C₁-C₂₀)-Alkyl, (C₂-C₂₀)-Alkenylresten ein oder mehrere einzelne -CH₂- oder-CH- Gruppen durch ―O- ersetzt sein können und wobei die Alkyl-, Cycloalkyl-, Alkenyl-, Alkinyl-, Aryl- und Heterozyklylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl; PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl,
N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
oder R1 und R2 bilden gemeinsam einen 3 bis 8 gliedrigen Aryl-, Cycloalkyl- oder Heterozyklyl-Ring, wobei der Aryl-, Cycloalkyl- oder Heterozyklyl-Ring substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂ und wobei der Aryl-, Cycloalkyl- oder Heterozyklyl-Ring mit einem weiteren Aryl-, Cycloalkyl- oder Heterozyklyl-Ring anneliert sein kann;
sowie derer physiologisch verträglichen Salze zur Herstellung eines Medikaments zur Behandlung des Metabolischen Syndroms.

2. Verwendung der Verbindungen der Formel I, gemäß Anspruch 1, **dadurch gekennzeichnet, dass** darin bedeuten
R1 H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch - O- ersetzt sein können und wobei die Alkyl-, Alkenyl- und Arylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON{(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl , SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
R2 H, OH, COOH, (C₁-C₈)-Alkyl, (C₃-C₈)-Cycloalkyl, (C₂-C₈)-Alkenyl, wobei in den (C₁-C₈)-Alkyl, oder (C₂-C₈)-Alkenylresten ein oder mehrere einzelne -CH₂- oder - CH- Gruppen durch -0- ersetzt sein können und wobei die Alkyl-, Cycloalkyl und Alkenylreste ein oder mehrfach substituiert sein können mit
F, Cl, Br, I, CF₃, NO₂, N₃, CN, =O, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl , SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂, , SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO_{2,} CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
oder R1 und R2 bilden gemeinsam einen 3 bis 8 gliedrigen Aryl-, Cycloalkyl- oder Heterozyklyl-Ring, wobei der Aryl-, Cycloalkyl- oder Heterozyklyl-Ring substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
sowie derer physiologisch verträglichen Salze zur Herstellung eines Medikaments zur Behandlung des Metabolischen Syndroms.

3. Verwendung der Verbindungen der Formel I, gemäß Anspruch 1, **dadurch gekennzeichnet, dass** darin bedeuten
R1 H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch-O- ersetzt sein können und wobei die Alkyl-, Alkenyl- und Arylreste ein oder mehrfach substituiert sein können mit
=O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
R2 H, (C₁-C₈)-Alkyl, (C₂-C₈)-Alkenyl;
sowie derer physiologisch verträglichen Salze zur Herstellung eines Medikaments zur Behandlung des Metabolischen Syndroms.

4. Verwendung der Verbindungen der Formel I, gemäß Anspruch 1, **dadurch gekennzeichnet, dass** darin bedeuten
R1 H, OH, (C₁-C₈)-Alkyl, (C₂-C₄)-Alkenyl, wobei in den (C₁-C₈)-Alkyl, (C₂-C₄)-Alkenylresten ein oder mehrere einzelne -CH₂- oder -CH- Gruppen durch -O-ersetzt sein können und wobei die Alkyl- und Alkenylreste ein oder mehrfach substituiert sein können mit
=O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
R2 H, (C₁-C₈)-Alkyl, (C₂-C₄)-Alkenyl;
sowie derer physiologisch verträglichen Salze zur Herstellung eines Medikaments zur Behandlung des Metabolischen Syndroms.

5. Verwendung der Verbindungen der Formel I, gemäß einem der Ansprüche 1 bis 4, sowie derer physiologisch verträglichen Salze zur Herstellung eines Medikaments zur Lipidsenkung im Plasma.

6. Verwendung der Verbindungen der Formel I, gemäß einem der Ansprüche 1 bis 4, sowie derer physiologisch verträglichen Salze zur Herstellung eines Medikaments zur Senkung der freien Fettsäuren (FFA) im Plasma.

7. Verwendung der Verbindungen der Formel I, gemäß einem der Ansprüche 1 bis 4, sowie derer physiologisch verträglichen Salze zur Herstellung eines Medikaments zur Senkung von Glycerol im Plasma.

8. Verwendung der Verbindungen der Formel I, gemäß einem der Ansprüche 1 bis 4, sowie derer physiologisch verträglichen Salze zur Herstellung eines Medikaments zur Senkung der Triglyceride im Plasma.

9. Verwendung der Verbindungen der Formel I, gemäß einem der Ansprüche 1 bis 4, sowie derer physiologisch verträglichen Salze zur Herstellung eines Medikaments zur Prophylaxe von Diabetes Typ 2.

10. Verwendung der Verbindungen der Formel I, gemäß einem der Ansprüche 1 bis 4, sowie derer physiologisch verträglichen Salze zur Herstellung eines Medikaments zur Behandlung der Diabetischen Dislipidämie.

11. Verwendung der Verbindungen der Formel I, gemäß einem der Ansprüche 1 bis 4, sowie derer physiologisch verträglichen Salze zur Herstellung eines Medikaments zur Behandlung der Obesitas.

12. Verwendung der Verbindungen der Formel I, worin R1 und R2 gleich Wasserstoff sind, gemäβ einem den Ansprüche 1-4.

13. Verbindungen der Formel I, 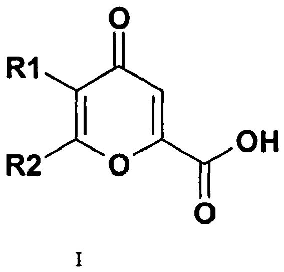 worin bedeuten
R1 (C₁-C₈)-Alkyl, (C₂-C₄)-Alkenyl, wobei die Alkyl- und Alkenylreste ein oder mehrfach mit F substituiert sein können;
R2 H;
sowie derer physiologisch verträglichen Salze.

14. Verbindungen der Formel I, gemäß Anspruch 13, **dadurch gekennzeichnet, dass** darin bedeuten
R1 (C₂-C₈)-Alkyl, wobei der Alkylrest ein oder mehrfach mit F substituiert sein kann;
R2 H;
sowie derer physiologisch verträglichen Salze.

15. Verwendung der Verbindungen der Formel I, gemäß Anspruch 1, **dadurch gekennzeichnet, dass** darin bedeuten
R1 H, OH, COOH, (C₁-C₈)-Alkyl, (C₂-C₈)-Alkenyl, Aryl, wobei in den (C₁-C₈)-Alkyl, (C₂-C₈)-Alkenylresten ein oder mehrere einzelne -CH₂- oder -CH- Gruppen durch - O- ersetzt sein können und wobei die Alkyl-, Alkenyl- und Arylreste ein oder mehrfach substituiert sein können mit
F, =O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂;
R2 H, (C₁-C₈)-Alkyl, (C₂-C₈)-Alkenyl;
sowie derer physiologisch verträglichen Salze zur Herstellung eines Medikaments zur Behandlung des Metabolischen Syndroms.

16. Verwendung der Verbindungen der Formel I, gemäß Anspruch 1, **dadurch gekennzeichnet, dass** darin bedeuten
R1 H, OH, (C₁-C₈)-Alkyl, (C₂-C₄)-Alkenyl, wobei in den (C₁-C₈)-Alkyl, (C₂-C₄)-Alkenylresten ein oder mehrere einzelne -CH₂- oder -CH- Gruppen durch -0-ersetzt sein können und wobei die Alkyl- und Alkenylreste ein oder mehrfach substituiert sein können mit
F, =O, Aryl, wobei der Aryl-Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH_{2;}
R2 H, (C₁-C₈)-Alkyl, (C₂-C₄)-Alkenyl;
sowie derer physiologisch verträglichen Salze zur Herstellung eines Medikaments zur Behandlung des Metabolischen Syndroms.

## Claims

1. The use of a compound of the formula I, 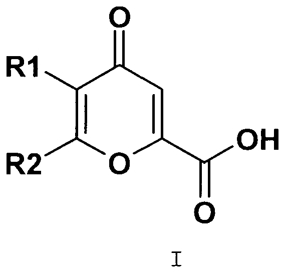 in which
R1 is H, F, Cl, Br, I, OH, CF₃, NO₂, CN, OCF₃, NH₂, NH(C₁-C₆)-alkyl, N ((C₁-C₆) -alkyl)₂, SF₅, SO₂-CH₃, COOH, COO- (C₁-C₆)-alkyl, CONH₂, (C₁-C₂₀)-alkyl, (C₃-C₂₀) -cycloalkyl, (C₂-C₂₀) -alkenyl, (C₂-C₂₀) - alkynyl, aryl, heterocycle, where in the (C₁-C₂₀)-alkyl and (C₂-C₂₀)-alkenyl radicals one or more individual -CH₂- or -CH- groups may be replaced by -O- and where the alkyl, cycloalkyl, alkenyl, alkynyl, aryl- and heterocyclyl radicals may be mono- or polysubstituted by
F, Cl, Br, I, CF₃, NO₂, N₃, CN, =O, COOH, COO(C₁-C₆)-alkyl, CONH₂, CONH(C₁-C₆)-alkyl, CON[(C₁-C₆)-alkyl]₂, cycloalkyl, (C₁-C₁₀) -alkyl, (C₂-C₆)-alkenyl, (C₂-C₆) -alkynyl, O- (C₁-C₆) - alkyl, O-CO-(C₁-C₆) -alkyl, O-CO-(C₁-C₆)-aryl, O-CO-(C₁-C₆)-heterocyclyl;
PO₃H₂, P(O)(Oalkyl)₂, (C₁-C₆)-alkylene-P(O)(Oalkyl)₂, O-P(O)(OH)₂, O-P(O)(Oalkyl)2, SO₃H, SO₂-NH₂, SO₂NH (C₁-C₆) -alkyl, SO₂N [(C₁-C₆)-alkyl]₂, S-(C₁-C₆)-alkyl, S-(CH₂)ₙ-aryl, S-(CH₂)ₙ-heterocyclyl, SO-(C₁-C₆)-alkyl, SO-(CH₂)ₙ-aryl, SO- (CH₂)ₙ-heterocyclyl, SO₂- (C₁-C₆)-alkyl, SO₂- (CH₂)ₙ-aryl, SO₂-(CH₂)ₙ-heterocyclyl, SO₂-NH (CH₂)ₙ-aryl, SO₂-NH (CH₂)ₙ-heterocyclyl, SO₂-N ((C₁-C₆) - alkyl) (CH₂)ₙ-aryl, SO₂-N ((C₁-C₆) -alkyl) (CH₂)ₙ-heterocyclyl, SO₂-N ((CH₂)ₙ-aryl)₂, SO₂-N((CH₂)ₙ-heterocyclyl)₂ where n = 0 - 6 and the aryl radical or heterocyclic radical may be substituted up to two times by F, Cl, Br, OH, CF₃, SF₅, NO₂, CN, OCF₃, O-(C₁-C₆)-alkyl, (C₁-C₆)-alkyl, NH₂; C(NH)(NH₂), NH₂, NH-(C₁-C₆)-alkyl, N((C₁-C₆)-alkyl)₂, NH(C₁-C₇)-acyl, NH-CO-(C₁-C₆)-alkyl, NH-COO-(C₁-C₆)-alkyl, NH-CO-aryl, NH-CO-heterocyclyl, NH-COO-aryl, NH-COO-heterocyclyl, NH-CO-NH-(C₁-C₆)-alkyl), NH-CO-NH-aryl, NH-CO-NH-heterocyclyl, N[(C₁-C₆) -alkyl] -CO- (C₁-C₆) -alkyl, N [(C₁-C₆) - alkyl] -COO- (C₁-C₆) -alkyl, N [(C₁-C₆) -alkyl] - CO-aryl, N[(C₁-C₆)-alkyl]-CO-heterocyclyl, N [(C₁-C₆) -alkyl] -COO-aryl, N [(C₁-C₆) -alkyl] - COO-heterocyclyl, N[(C₁-C₆)-alkyl]-CO-NH-(C₁-C₆)-alkyl), N [(C₁-C₆) -alkyl] -CO-NH-aryl, N[(C₁-C₆)-alkyl] -CO-NH-heterocyclyl, N[(C₁-C₆) -alkyl] -CO-N ((C₁-C₆) -alkyl)₂, N [(C₁-C₆) - alkyl]-CO-N((C₁-C₆) -alkyl) -aryl, N [(C₁-C₆) - alkyl]-CO-N((C₁-C₆)-alkyl)-heterocyclyl, N [(C₁-C₆) -alkyl] -CO-N (aryl)₂, N [(C₁-C₆) - alkyl]-CO-N (heterocyclyl)₂, N(aryl)-CO-(C₁-C₆)-alkyl, N(heterocyclyl)-CO-(C₁-C₆)-alkyl, N(aryl)-COO-(C₁-C₆)-alkyl, N(heterocyclyl)-COO-(C₁-C₆)-alkyl, N(aryl)-CO-aryl, N(heterocyclyl)-CO-aryl, N(aryl)-COO-aryl, N(heterocyclyl)-COO-aryl, N(aryl)-CO-NH-(C₁-C₆)-alkyl, N(heterocyclyl)-CO-NH-(C₁-C₆)-alkyl, N(aryl)-CO-NH-aryl, N(heterocyclyl)-CO-NH-aryl, N (aryl) -CO-N ((C₁-C₆) -alkyl)₂, N (heterocyclyl) -CO-N ((C₁-C₆) -alkyl)₂, N(aryl)-CO-N[(C₁-C₆)-alkyl]-aryl, N(heterocyclyl)-CO-N[(C₁-C₆)-alkyl]-aryl, N(aryl)-CO-N(aryl)₂, N(heterocyclyl)-CON(aryl)₂, aryl, 0- (CH₂)ₙ-aryl, O- (CH₂)ₙ-heterocyclyl, where n = 0 - 6 and where the aryl or heterocyclyl radical may be mono-to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
R2 is H, F, Cl, Br, I, OH, CF₃, NO₂, CN, OCF₃, NH₂, NH(C₁-C₆)-alkyl, N ((C₁-C₆) -alkyl)₂, SF₅, SO₂-CH₃, COOH, COO-(C₁-C₆)-alkyl, CONH₂, (C₁-C₂₀)-alkyl, (C₃-C₂₀) -cycloalkyl, (C₂-C₂₀) -alkenyl, (C₂-C₂₀) - alkynyl, aryl, heterocycle, where in the (C₁-C₂₀)-alkyl and (C₂-C₂₀)-alkenyl radicals one or more individual -CH₂- or -CH- groups may be replaced by -O- and where the alkyl, cycloalkyl, alkenyl, alkynyl, aryl and heterocycle radicals may be mono- or polysubstituted by
F, Cl, Br, I, CF₃, NO₂, N₃, CN, =O, COOH, COO(C₁-C₆)-alkyl, CONH₂, CONH(C₁-C₆)-alkyl, CON[(C₁-C₆)-alkyl]₂, cycloalkyl, (C₁-C₁₀)-alkyl, (C₂-C₆)-alkenyl, (C₂-C₆)-alkynyl, O-(C₁-C₆)-alkyl, O-CO-(C₁-C₆)-alkyl, O-CO-(C₁-C₆)-aryl, O-CO-(C₁-C₆)-heterocyclyl;
PO₃H₂, P(O)(Oalkyl)₂, (C₁-C₆)-alkylene-P(O)(Oalkyl)₂, O-P(O)(OH)₂, O-P(O)(Oalkyl)₂, SO₃H, SO₂-NH₂, SO₂NH (C₁-C₆) -alkyl, SO₂N[(C₁-C₆)-alkyl]₂, S-(C₁-C₆)-alkyl, S- (CH₂)ₙ-aryl, S-(CH₂)ₙ-heterocyclyl, SO-(C₁-C₆)-alkyl, SO-(CH₂)ₙ-aryl, SO-(CH₂)ₙ-heterocyclyl, SO₂-(C₁-C₆)-alkyl, SO₂-(CH₂)ₙ-aryl, SO₂-(CH₂)ₙ-heterocyclyl, SO₂-NH (CH₂)ₙ-aryl, SO₂-NH (CH₂)ₙ-heterocyclyl, SO₂-N((C₁-C₆)-alkyl) (CH₂)ₙ-aryl, SO₂-N((C₁-C₆)-alkyl) (CH₂)ₙ-heterocyclyl, SO₂-N((CH₂)ₙ-aryl)₂, SO₂-N((CH₂)ₙ-heterocyclyl)₂ where n = 0 - 6 and the aryl radical or heterocyclic radical may be substituted up to two times by F, Cl, Br, OH, CF₃, SF₅, NO₂, CN, OCF₃, O-(C₁-C₆)-alkyl, (C₁-C₆)-alkyl, NH₂; C(NH)(NH₂), NH₂, NH-(C₁-C₆)-alkyl, N((C₁-C₆)-alkyl)₂, NH(C₁-C₇)-acyl, NH-CO-(C₁-C₆)-alkyl, NH-COO- (C₁-C₆) -alkyl, NH-CO-aryl, NH-CO-heterocyclyl, NH-COO-aryl, NH-COO-heterocyclyl, NH-CO-NH-(C₁-C₆)-alkyl), NH-CO-NH-aryl, NH-CO-NH-heterocyclyl, N[(C₁-C₆) -alkyl] -CO- (C₁-C₆) -alkyl, N [(C₁-C₆)-alkyl] -COO- (C₁-C₆) -alkyl, N [(C₁-C₆)-alkyl]-CO-aryl, N[(C₁-C₆)-alkyl]-CO-heterocyclyl, N[(C₁-C₆)-alkyl]-COO-aryl, N [(C₁-C₆) -alkyl]-COO-heterocyclyl, N[(C₁-C₆)-alkyl]-CO-NH-(C₁-C₆)-alkyl), N [(C₁-C₆) -alkyl] -CO-NH-aryl, N[(C₁-C₆)-alkyl]-CO-NH-heterocyclyl, N[(C₁-C₆) -alkyl] -CO-N ((C₁-C₆) -alkyl)₂, N [(C₁-C₆)-alkyl] -CO-N ((C₁-C₆) -alkyl) -aryl, N [(C₁-C₆)-alkyl]-CO-N((C₁-C₆)-alkyl)-heterocyclyl, N[(C₁-C₆)-alkyl]-CO-N(aryl)₂, N[(C₁-C₆)-alkyl]-CO-N(heterocyclyl)₂, N(aryl)-CO-(C₁-C₆)-alkyl, N(heterocyclyl)-CO-(C₁-C₆)-alkyl, N(aryl)-COO-(C₁-C₆)-alkyl, N(heterocyclyl)-COO-(C₁-C₆)-alkyl, N(aryl)-CO-aryl, N(heterocyclyl)-CO-aryl, N(aryl)-COO-aryl, N(heterocyclyl)-COO-aryl, N(aryl)-CO-NH-(C₁-C₆)-alkyl, N(heterocyclyl)-CO-NH-(C₁-C₆)-alkyl, N(aryl)-CO-NH-aryl, N(heterocyclyl)-CO-NH-aryl, N (aryl) -CO-N ((C₁-C₆) -alkyl)₂, N(heterocyclyl)-CO-N((C₁-C₆)-alkyl)₂, N(aryl)-CO-N[(C₁-C₆)-alkyl]-aryl, N(heterocyclyl)-CO-N[(C₁-C₆)-alkyl]-aryl, N(aryl)-CO-N(aryl)₂, N(heterocyclyl)-CON(aryl)₂, aryl, O-(CH₂)ₙ-aryl, O-(CH₂)ₙ-heterocyclyl, where n = 0 - 6 and where the aryl or heterocyclyl radical may be mono-to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
or R1 and R2 together form a 3- to 8-membered aryl, cycloalkyl or heterocyclyl ring, where the aryl, cycloalkyl or heterocyclyl ring may be substituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO- (C₁-C₆)-alkyl, CONH₂ and where the aryl, cycloalkyl or heterocyclyl ring may be fused to a further aryl, cycloalkyl or heterocyclyl ring;
and physiologically acceptable salts thereof, for producing a medicament for the treatment of the metabolic syndrome.

2. The use of a compound of the formula I as claimed in claim 1 wherein
R1 is H, OH, COOH, (C₁-C₈) -alkyl, (C₂-C₈) -alkenyl, aryl, where in the (C₁-C₈)-alkyl and (C₂-C₈)-alkenyl radicals one or more individual -CH₂- or -CH- groups may be replaced by -O- and where the alkyl, alkenyl and aryl radicals may be mono- or polysubstituted by
F, Cl, Br, I, CF₃, NO₂, N₃, CN, =O, COOH, COO (C₁-C₆) -alkyl, CONH₂, CONH (C₁-C₆) -alkyl, CON [(C₁-C₆)-alkyl]₂, cycloalkyl, (C₁-C₁₀)-alkyl, (C₂-C₆) -alkenyl, (C₂-C₆) -alkynyl, O-(C₁-C₆)-alkyl, O-CO-(C₁-C₆)-alkyl, O-CO-(C₁-C₆)-aryl, O-CO-(C₁-C₆)-heterocyclyl;
PO₃H₂, P(O)(Oalkyl)₂, (C₁-C₆) -alkylene-P(O) (Oalkyl)₂, O-P(O) (OH)₂, O-P(O) (Oalkyl) 2, SO₃H, SO₂-NH₂, SO₂NH (C₁-C₆) -alkyl, SO₂N[(C₁-C₆)-alkyl]₂, S-(C₁-C₆)-alkyl, S-(CH₂)ₙ-aryl, S-(CH₂)ₙ-heterocyclyl, SO-(C₁-C₆)-alkyl, SO-(CH₂)ₙ-aryl, SO-(CH₂)ₙ-heterocyclyl, SO₂-(C₁-C₆) -alkyl, SO₂-(CH₂)ₙ-aryl, SO₂-(CH₂)ₙ-heterocyclyl, SO₂-NH (CH₂)ₙ-aryl, SO₂-NH (CH₂)ₙ-heterocyclyl, SO₂-N((C₁-C₆)-alkyl) (CH₂)ₙ-aryl, SO₂-N((C₁-C₆)-alkyl) (CH₂)ₙ-heterocyclyl, SO₂-N((CH₂)ₙ-aryl)₂, SO₂-N((CH₂)ₙ-(heterocyclyl)₂ where n = 0 - 6 and the aryl radical or heterocyclic radical may be substituted up to two times by F, Cl, Br, OH, CF₃, SF₅, NO₂, CN, OCF₃, O-(C₁-C₆)-alkyl, (C₁-C₆)-alkyl, NH₂;
C (NH) (NH₂), NH₂, NH-(C₁-C₆)-alkyl, N((C₁-C₆)-alkyl)₂, NH(C₁-C₇)-acyl, NH-CO-(C₁-C₆)-alkyl, NH-COO-(C₁₋C₆)-alkyl, NH-CO-aryl, NH-CO-heterocyclyl, NH-COO-aryl, NH-COO-heterocyclyl, NH-CO-NH-(C₁-C₆)-alkyl), NH-CO-NH-aryl, NH-CO-NH-heterocyclyl, N[(C₁-C₆) -alkyl] -CO- (C₁-C₆) -alkyl, N [(C₁-C₆)-alkyl] -COO-(C₁-C₆)-alkyl, N [(C₁-C₆)-alkyl]-CO-aryl, N [(C₁-C₆)-alkyl]-CO-heterocyclyl, N[(C₁-C₆)-alkyl]-COO-aryl, N [(C₁-C₆)-alkyl]-COO-heterocyclyl, N[(C₁-C₆)-alkyl]-CO-NH-(C₁-C₆)-alkyl), N [(C₁-C₆)-alkyl]-CO-NH-aryl, N[(C₁-C₆)-alkyl]-CO-NH-heterocyclyl, N[(C₁-C₆)-alkyl]-CO-N((C₁-C₆)-alkyl)₂, N[(C₁-C₆)-alkyl]-CO-N ((C₁-C₆)-alkyl) -aryl, N [(C₁-C₆)-alkyl]-CO-N((C₁-C₆)-alkyl)-heterocyclyl, N [(C₁-C₆)-alkyl]-CO-N(aryl)₂, N[(C₁-C₆)-alkyl]-CO-N(heterocyclyl)₂, N(aryl)-CO-(C₁-C₆)-alkyl, N(heterocyclyl)-CO-(C₁-C₆)-alkyl, N(aryl)-COO-(C₁-C₆)-alkyl, N(heterocyclyl)-COO-(C₁-C₆)-alkyl, N(aryl)-CO-aryl, N(heterocyclyl)-CO-aryl, N(aryl)-COO-aryl, N(heterocyclyl)-COO-aryl, N(aryl)-CO-NH-(C₁-C₆)-alkyl, N(heterocyclyl)-CO-NH-(C₁-C₆)-alkyl, N(aryl)-CO-NH-aryl, N(heterocyclyl)-CO-NH-aryl, N (aryl) -CO-N ((C₁-C₆)-alkyl)₂, N (heterocyclyl) -CO-N ((C₁-C₆)-alkyl)₂, N (aryl) -CO-N [(C₁-C₆)-alkyl] -aryl,
N(heterocyclyl)-CO-N[(C₁-C₆)-alkyl]-aryl, N(aryl)-CO-N(aryl)₂, N(heterocyclyl)-CON(aryl)₂, aryl, O-(CH₂)ₙ-aryl, O-(CH₂)ₙ-heterocyclyl, where n = 0 - 6 and where the aryl or heterocyclyl radical may be mono-to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
R2 is H, OH, COOH, (C₁-C₈)-alkyl, (C₃-C₈)-cycloalkyl, (C₂-C₈)-alkenyl, where in the (C₁-C₈)-alkyl and (C₂-C₈)-alkenyl radicals one or more individual -CH₂- or -CH- groups may be replaced by -0- and where the alkyl, cycloalkyl and alkenyl radicals may be mono- or polysubstituted by
F, Cl, Br, I, CF₃, NO₂, N₃, CN, =O, COOH, COO(C₁-C₆)-alkyl, CONH₂, CONH(C₁-C₆)-alkyl, CON[(C₁-C₆)-alkyl]₂, cycloalkyl, (C₁-C₁₀)-alkyl, (C₂-C₆)-alkenyl, (C₂-C₆)-alkynyl, O-(C₁-C₆)-alkyl O-CO-(C₁-C₆)-alkyl, O-CO-(C₁-C₆)-aryl, O-CO-(C₁-C₆)-heterocyclyl;
PO₃H₂, P(O)(Oalkyl)₂, (C₁-C₆)-alkylene-P(O)(Oalkyl)₂, O-P(O)(OH)₂, O-P(O)(Oalkyl)2, SO₃H, SO₂-NH₂, SO₂NH (C₁-C₆) -alkyl, SO₂N[(C₁-C₆)-alkyl]₂, S-(C₁-C₆)-alkyl, S-(CH₂)ₙ-aryl, S-(CH₂)ₙ-heterocyclyl, SO-(C₁-C₆)-alkyl, SO-(CH₂)ₙ-aryl, SO-(CH₂)ₙ-heterocyclyl, SO₂-(C₁-C₆)-alkyl, SO₂-(CH₂)ₙ-aryl, SO₂-(CH₂)ₙ-heterocyclyl, SO₂-NH (CH₂)ₙ-aryl, SO₂-NH (CH₂)ₙ-heterocyclyl, SO₂-N((C₁-C₆)-alkyl) (CH₂)ₙ-aryl, SO₂-N((C₁-C₆)-alkyl)(CH₂)ₙ-heterocyclyl, SO₂-N((CH₂)ₙ-aryl)₂, SO₂-N((CH₂)ₙ-(heterocyclyl)₂ where n = 0 - 6 and the aryl radical or heterocyclic radical may be substituted up to two times by F, Cl, Br, OH, CF₃, SF₅, NO₂, CN, OCF₃, O-(C₁-C₆)-alkyl, (C₁-C₆)-alkyl, NH₂; C (NH) (NH₂), NH₂, NH- (C₁-C₆) -alkyl, N((C₁-C₆)-alkyl)₂, NH(C₁-C₇-acyl, NH-CO-(C₁-C₆)-alkyl, NH-COO- (C₁-C₆)-alkyl, NH-CO-aryl, NH-CO-heterocyclyl, NH-COO-aryl, NH-COO-heterocyclyl, NH-CO-NH-(C₁-C₆)-alkyl), NH-CO-NH-aryl, NH-CO-NH-heterocyclyl, N[(C₁-C₆)-alkyl]-CO- (C₁-C₆)-alkyl, N[(C₁-C₆)-alkyl] -COO-(C₁-C₆)-alkyl, N[(C₁-C₆)-alkyl]-CO-aryl, N[(C₁-C₆)-alkyl]-CO-heterocyclyl, N[(C₁-C₆)-alkyl] -COO-aryl, N[(C₁-C₆)-alkyl]-COO-heterocyclyl, N[(C₁-C₆)-alkyl]-CO-NH-(C₁-C₆)-alkyl), N[(C₁-C₆) -alkyl] -CO-NH-aryl, N[(C₁-C₆)-alkyl]-CO-NH-heterocyclyl, N[(C₁-C₆)-alkyl]-CO-N ((C₁-C₆)-alkyl)₂, N[(C₁-C₆)-alkyl]-CO-N((C₁-C₆)-alkyl)-aryl, N[(C₁-C₆)-alkyl]-CO-N((C₁-C₆)-alkyl)-heterocyclyl, N[(C₁-C₆)-alkyl]-CO-N (aryl)₂, N[(C₁-C₆)-alkyl]-CO-N(heterocyclyl)₂, N(aryl)-CO-(C₁-C₆)-alkyl, N(heterocyclyl)-CO-(C₁-C₆)-alkyl, N(aryl)-COO-(C₁-C₆)-alkyl, N(heterocyclyl)-COO-(C₁-C₆)-alkyl, N(aryl)-CO-aryl, N(heterocyclyl)-CO-aryl, N(aryl)-COO-aryl, N(heterocyclyl)-COO-aryl, N(aryl)-CO-NH-(C₁-C₆)-alkyl, N(heterocyclyl)-CO-NH-(C₁-C₆)-alkyl, N(aryl)-CO-NH-aryl, N(heterocyclyl)-CO-NH-aryl, N (aryl) -CO-N ((C₁-C₆)-alkyl)₂, N (heterocyclyl) -CO-N ((C₁-C₆)-alkyl)₂, N(aryl)-CO-N[(C₁-C₆)-alkyl]-aryl, N(heterocyclyl)-CO-N[(C₁-C₆)-alkyl]-aryl, N(aryl)-CO-N(aryl)₂, N(heterocyclyl)-CON(aryl)₂, aryl, 0- (CH₂)ₙ-aryl, O-(CH₂)ₙ-heterocyclyl, where n = 0 - 6, where the aryl or heterocyclyl radical may be mono-to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
or R1 and R2 together form a 3- to 8-membered aryl, cycloalkyl or heterocyclyl ring, where the aryl, cycloalkyl or heterocyclyl ring may be substituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO- (C₁-C₆) -alkyl, CONH₂;
and physiologically acceptable salts thereof, for producing a medicament for the treatment of the metabolic syndrome.

3. The use of a compound of the formula I as claimed in claim 1 wherein
R1 is H, OH, COOH, (C₁-C₈)-alkyl, (C₂-C₈)-alkenyl, aryl, where in the (C₁-C₈)-alkyl and (C₂-C₈)-alkenyl radicals one or more individual -CH₂- or -CH- groups may be replaced by -O- and where the alkyl, alkenyl and aryl radicals may be mono- or polysubstituted by
=O, aryl, where the aryl radical may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
R2 is H, (C₁-C₈)-alkyl, (C₂-C₈)-alkenyl;
and physiologically acceptable salts thereof, for producing a medicament for the treatment of the metabolic syndrome.

4. The use of a compound of the formula I as claimed in claim 1 wherein
R1 is H, OH, (C₁-C₈)-alkyl, (C₂-C₄)-alkenyl, where in the (C₁-C₈)-alkyl and (C₂-C₄)-alkenyl radicals one or more individual -CH₂- or -CH- groups may be replaced by -O- and where the alkyl and alkenyl radicals may be mono- or polysubstituted by
=O, aryl, where the aryl radical may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
R2 is H, (C₁-C₈)-alkyl, (C₂-C₄)-alkenyl;
and physiologically acceptable salts thereof, for producing a medicament for the treatment of the metabolic syndrome.

5. The use of a compound of the formula I as claimed in any of claims 1 to 4 and of the physiologically acceptable salts thereof for producing a medicament for reducing plasma lipids.

6. The use of a compound of the formula I as claimed in any of claims 1 to 4 and of the physiologically acceptable salts thereof for producing a medicament for reducing plasma free fatty acids (FFA).

7. The use of a compound of the formula I as claimed in any of claims 1 to 4 and of the physiologically acceptable salts thereof for producing a medicament for reducing plasma glycerol.

8. The use of a compound of the formula I as claimed in any of claims 1 to 4 and of the physiologically acceptable salts thereof for producing a medicament for reducing plasma triglycerides.

9. The use of a compound of the formula I as claimed in any of claims 1 to 4 and of the physiologically acceptable salts thereof for producing a medicament for the prophylaxis of type 2 diabetes.

10. The use of a compound of the formula I as claimed in any of claims 1 to 4 and of the physiologically acceptable salts thereof for producing a medicament for the treatment of diabetic dyslipidemia.

11. The use of a compound of the formula I as claimed in any of claims 1 to 4 and of the physiologically acceptable salts thereof for producing a medicament for the treatment of obesity.

12. The use of a compound of the formula I as claimed in any of claims 1-4 in which R1 and R2 are hydrogen as a medicament.

13. A compound of the formula I 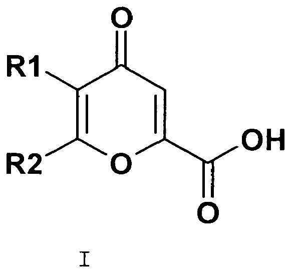 in which
R1 is (C₁-C₈)-alkyl or (C₂-C₄)-alkenyl, where the alkyl and alkenyl radicals may be mono- or polysubstituted by F;
R2 is H;
and physiologically acceptable salts thereof.

14. The compound of the formula I as claimed in claim 13 wherein
R1 is (C₂-C₈)-alkyl, where the alkyl radical may be mono- or polysubstituted by F;
R2 is H;
and physiologically acceptable salts thereof.

15. The use of a compound of the formula I as claimed in claim 1 wherein
R1 is H, OH, COOH, (C₁-C₈)-alkyl, (C₂-C₈)-alkenyl, aryl, where in the (C₁-C₈)-alkyl and (C₂-C₈)-alkenyl radicals one or more individual -CH₂- or -CH- groups may be replaced by -O- and where the alkyl, alkenyl and aryl radicals may be mono- or polysubstituted by
F, =O, aryl, where the aryl radical may be mono- to trisubstituted by F, Cl, Br, I, OH, CF₃, NO₂, CN, OCF₃, O-(C₁-C₆)-alkyl, (C₁-C₆)-alkyl, NH₂, NH (C₁-C₆) -alkyl, N((C₁-C₆)-alkyl)₂, SF₅, SO₂-CH₃, COOH, COO-(C₁-C₆)-alkyl, CONH₂;
R2 is H, (C₁-C₈)-alkyl, (C₂-C₈)-alkenyl;
and physiologically acceptable salts thereof, for producing a medicament for the treatment of the metabolic syndrome.

16. The use of a compound of the formula I as claimed in claim 1 wherein
R1 is H, OH, (C₁-C₈)-alkyl, (C₂-C₄)-alkenyl, where in the (C₁-C₈)-alkyl and (C₂-C₄)-alkenyl radicals one or more individual -CH₂- or -CH- groups may be replaced by -O- and where the alkyl and alkenyl radicals may be mono- or polysubstituted by
F, =O, aryl, where the aryl radical may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SF₅, SO₂-CH₃, COOH, COO-(C₁-C₆)-alkyl, CONH₂;
R2 is H, (C₁-C₈)-alkyl, (C₂-C₄)-alkenyl;
and physiologically acceptable salts thereof, for producing a medicament for the treatment of the metabolic syndrome.

## Revendications

1. Utilisation des composés de formule I 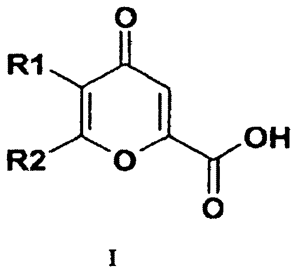 dans laquelle
R1 signifie H, F, Cl, Br, I, OH, CF₃, NO₂, CN, OCF₃, NH₂, NH-alkyle en (C₁-C₆), N-(alkyle en (C₁-C₆))₂, SF₅, SO₂-CH₃, COOH, COO-alkyle en (C₁-C₆), CONH₂, alkyle en (C₁-C₂₀), cycloalkyle en (C₃-C₂₀), alcényle en (C₂-C₂₀), alcynyle en (C₂-C₂₀), aryle, hétérocycle, un ou plusieurs groupes -CH₂ ou -CH- individuels dans les radicaux alkyle en (C₁-C₂₀), alcényle en (C₂-C₂₀) pouvant être remplacés par -0- et les radicaux alkyle, cycloalkyle, alcényle, alcynyle, aryle et hétérocyclyle pouvant être substitués une ou plusieurs fois avec
F, Cl, Br, I, CF₃, NO₂, N₃, CN, =O, COOH, COO-alkyle en (C₁-C₆), CONH₂, CONH-alkyle en (C₁-C₆), CON[alkyle en (C₁-C₆)]₂, cycloalkyle, alkyle en (C₁-C₁₀), alcényle en (C₂-C₆), alcynyle en (C₂-C₆), 0-alkyle en (C₁-C₆), O-CO-alkyle en (C₁-C₆), O-CO-aryle en (C₁-C₆), O-CO-hétérocyclyle en (C₁-C₆) ;
PO₃H₂, P(O)(O-alkyle)₂, alkylène en (C₁-C₆)-P(O)(O-alkyle)₂, O-P(O)(OH)₂, O-P(O)(O-alkyle)₂, SO₃H, SO₂-NH₂, SO₂NH-alkyle en (C₁-C₆), SO₂N-[alkyle en (C₁-C₆)]₂, S-alkyle en (C₁-C₆), S-(CH₂)ₙ-aryle, S-(CH₂)ₙ-hétérocyclyle, SO-alkyle en (C₁-C₆), SO-(CH₂)ₙ-aryle, SO-(CH₂)ₙ-hétérocyclyle, SO₂-alkyle en (C₁-C₆), SO₂-(CH₂)ₙ-aryle, SO₂-(CH₂)ₙ-hétérocyclyle, SO₂-NH(CH₂)ₙ-aryle, SO₂-NH(CH₂)ₙ-hétérocyclyle, SO₂-N (alkyle en (C₁-C₆))(CH₂)ₙ-aryle, SO₂-N(alkyle en (C₁-C₆)) (CH₂)ₙ-hétérocyclyle, SO₂-N((CH₂)ₙ-aryle)₂, SO₂-N((CH₂)ₙ-(hétérocyclyle)₂, n pouvant être 0 à 6 et le radical aryle ou le radical hétérocyclique pouvant être substitué jusqu'à deux fois avec F, Cl, Br, OH, CF₃, SF₅, NO₂, CN, OCF₃, O-alkyle en (C₁-C₆), alkyle en (C₁-C₆), NH₂ ;
C(NH)(NH₂), NH₂, NH-alkyle en (C₁-C₆), N(alkyle en (C₁-C₆))₂, NH-acyle en (C₁-C₇), NH-CO-alkyle en (C₁-C₆), NH-COO-alkyle en (C₁-C₆), NH-CO-aryle, NH-CO-hétérocyclyle, NH-COO-aryle, NH-COO-hétérocyclyle, NH-CO-NH-alkyle en (C₁-C₆)), NH-CO-NH-aryle, NH-CO-NH-hétérocyclyle, N[alkyle en (C₁-C₆)]-CO-alkyle en (C₁-C₆), N [alkyle en (C₁-C₆)]-COO-alkyle en (C₁-C₆), N [alkyle en (C₁-C₆)]-CO-aryle, N [alkyle en (C₁-C₆)]-CO-hétérocyclyle, N[alkyle en (C₁-C₆)]-COO-aryle, N [alkyle en (C₁-C₆)]-COO-hétérocyclyle, N [alkyle en (C₁-C₆)]-CO-NH-alkyle en (C₁-C₆)), N [alkyle en (C₁-C₆)]-CO-NH-aryle, N[alkyle en (C₁-C₆)]-CO-NH-hétérocyclyle, N [alkyle en (C₁-C₆)]-CO-N(alkyle en (C₁-C₆))₂, N[alkyle en (C₁-C₆)]-CO-N(alkyle en (C₁-C₆))-aryle, N[alkyle en (C₁-C₆)]-CO-N(alkyle en (C₁-C₆))-hétérocyclyle, N[alkyle en (C₁-C₆)]-CON(aryle)₂, N[alkyle en (C₁-C₆)]-CON(hétérocyclyle)₂, N(aryle)-CO-alkyle en (C₁-C₆), N(hétérocyclyle)-CO-alkyle en (C₁-C₆), N(aryle)-COO-alkyle en (C₁-C₆), N(hétérocyclyle)-COO-alkyle en (C₁-C₆), N(aryle)-CO-aryle, N(hétérocyclyle)-CO-aryle, N(aryle)-COO-aryle, N(hétérocyclyle)-COO-aryle, N(aryle)-CO-NH-alkyle en (C₁-C₆), N(hétérocyclyle)-CO-NH-alkyle en (C₁-C₆), N(aryle)-CO-NH-aryle, N(hétérocyclyle)-CO-NH-aryle, N(aryle)-CO-N(alkyle en (C₁-C₆))₂, N(hétérocyclyle)-CO-N(alkyle en (C₁-C₆))₂, N(aryle)-CO-N[alkyle en (C₁-C₆)]-aryle, N(hétérocyclyle)-CO-N[alkyle en (C₁-C₆)]-aryle, N(aryle)-CO-N(aryle)₂, N(hétérocyclyle)-CON(aryle)₂, aryle, O-(CH₂)ₙ-aryle, O- (CH₂)ₙ-hétérocyclyle, n pouvant être 0 à 6, le radical aryle ou hétérocyclyle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH₂;
R2 signifie H, F, Cl, Br, I, OH, CF₃, NO₂, CN, OCF₃, NH₂, NH-alkyle en (C₁-C₆), N (alkyle en (C₁-C₆))₂, SF₅, SO₂-CH₃, COOH, COO-alkyle en (C₁-C₆), CONH₂, alkyle en (C₁-C₂₀), cycloalkyle en (C₃-C₂₀), alcényle en (C₂-C₂₀), alcynyle en (C₂-C₂₀), aryle, hétérocycle, un ou plusieurs groupes -CH₂ ou -CH- individuels dans les radicaux alkyle en (C₁-C₂₀), alcényle en (C₂-C₂₀) pouvant être remplacés par -O- et les radicaux alkyle, cycloalkyle, alcényle, alcynyle, aryle et hétérocyclyle pouvant être substitués une ou plusieurs fois avec
F, Cl, Br, I, CF₃, NO₂, N₃, CN, =O, COOH, COO-alkyle en (C₁-C₆), CONH₂, CONH-alkyle en (C₁-C₆), CON[alkyle en (C₁-C₆)]₂, cycloalkyle, alkyle en (C₁-C₁₀), alcényle en (C₂-C₆), alcynyle en (C₂-C₆), 0-alkyle en (C₁-C₆), O-CO-alkyle en (C₁-C₆), O-CO-aryle en (C₁-C₆), O-CO-hétérocyclyle en (C₁-C₆) ; PO₃H₂, P(O) (O-alkyle)₂, alkylène en (C₁-C₆)-P(O)(O-alkyle)₂, O-P(O)(OH)₂, O-P(O)(O-alkyle)2, SO₃H, SO₂-NH₂, SO₂NH-alkyle en (C₁-C₆), SO₂N-[alkyle en (C₁-C₆)]₂, S-alkyle en (C₁-C₆), S-(CH₂)ₙ-aryle, S-(CH₂)ₙ-hétérocyclyle, SO-alkyle en (C₁-C₆), SO-(CH₂)ₙ-aryle, SO-(CH₂)ₙ-hétérocyclyle, SO₂-alkyle en (C₁-C₆), SO₂-(CH₂)ₙ-aryle, SO₂-(CH₂)ₙ-hétérocyclyle, SO₂-NH(CH₂)ₙ-aryle, SO₂-NH(CH₂)ₙ-hétérocyclyle, SO₂-N (alkyle en (C₁-C₆)) (CH₂)ₙ-aryle, SO₂-N(alkyle en (C₁-C₆)) (CH₂)ₙ-hétérocyclyle, SO₂-N((CH₂)ₙ-aryle)₂, SO₂-N((CH₂)ₙ-(hétérocyclyle)₂, n pouvant être 0 à 6 et le radical aryle ou le radical hétérocyclique pouvant être substitué jusqu'à deux fois avec F, Cl, Br, OH, CF₃, SF₅, NO₂, CN, OCF₃, O-alkyle en (C₁-C₆), alkyle en (C₁-C₆), NH₂ ;
C(NH)(NH₂), NH₂, NH-alkyle en (C₁-C₆), N (alkyle en (C₁-C₆))₂, NH-acyle en (C₁-C₇), NH-CO-alkyle en (C₁-C₆), NH-COO-alkyle en (C₁-C₆), NH-CO-aryle, NH-CO-hétérocyclyle, NH-COO-aryle, NH-COO-hétérocyclyle, NH-CO-NH-alkyle en (C₁-C₆)), NH-CO-NH-aryle, NH-CO-NH-hétérocyclyle, N[alkyle en (C₁-C₆)]-CO-alkyle en (C₁-C₆), N [alkyle en (C₁-C₆)]-COO-alkyle en (C₁-C₆), N [alkyle en (C₁-C₆)]-CO-aryle, N [alkyle en (C₁-C₆)]-CO-hétérocyclyle, N[alkyle en (C₁-C₆)]-COO-aryle, N[alkyle en (C₁-C₆)]-COO-hétérocyclyle, N[alkyle en (C₁-C₆)]-CO-NH-alkyle en (C₁-C₆)), N [alkyle en (C₁-C₆)] -CO-NH-aryle, N [alkyle en (C₁-C₆)]-CO-NH-hétérocyclyle, N[alkyle en (C₁-C₆)]-CO-N(alkyle en (C₁-C₆))₂, N[alkyle en (C₁-C₆)]-CO-N(alkyle en (C₁-C₆))-aryle, N[alkyle en (C₁-C₆)]-CO-N(alkyle en (C₁-C₆)) -hétérocyclyle, N[alkyle en (C₁-C₆)]-CON(aryle)₂, N [alkyle en (C₁-C₆)]-CON(hétérocyclyle)₂, N(aryle)-CO-alkyle en (C₁-C₆), N(hétérocyclyle)-CO-alkyle en (C₁-C₆), N(aryle)-COO-alkyle en (C₁-C₆), N(hétérocyclyle)-COO-alkyle en (C₁-C₆), N(aryle)-CO-aryle, N(hétérocyclyle)-CO-aryle, N(aryle)-COO-aryle, N(hétérocyclyle)-COO-aryle, N(aryle)-CO-NH-alkyle en (C₁-C₆), N(hétérocyclyle)-CO-NH-alkyle en (C₁-C₆), N(aryle)-CO-NH-aryle, N(hétérocyclyle)-CO-NH-aryle, N(aryle)-CO-N(alkyle en (C₁-C₆))_{2,} N(hétérocyclyle)-CO-N(alkyle en (C₁-C₆))₂, N(aryle)-CO-N[alkyle en (C₁-C₆)]-aryle, N(hétérocyclyle)-CO-N[alkyle en (C₁-C₆)]-aryle, N(aryle)-CO-N(aryle)₂, N(hétérocyclyle)-CON(aryle)₂, aryle, 0- (CH₂)ₙ-aryle, O- (CH₂)ₙ-hétérocyclyle, n pouvant être 0 à 6, le radical aryle ou hétérocyclyle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁₋C₆), N(alkyle en (C₁-C₆))₂, SF₅, SO₂-CH₃, COOH, COO-alkyle en (C₁-C₆), CONH₂ ;
ou R1 et R2 forment ensemble un cycle aryle, cycloalkyle ou hétérocyclyle de 3 à 8 éléments, le cycle aryle, cycloalkyle ou hétérocyclyle pouvant être substitué avec F, Cl, Br, I, OH, CF₃, NO₂, CN, OCF₃, 0-alkyle en (C₁-C₆), alkyle en (C₁-C₆), NH₂, NH-alkyle en (C₁-C₆), N(alkyle en (C₁-C₆))₂, SF₅, SO₂-CH₃, COOH, COO-alkyle en (C₁-C₆), CONH₂ et le cycle aryle, cycloalkyle ou hétérocyclyle pouvant être annelé avec un autre cycle aryle, cycloalkyle ou hétérocyclyle ;
ainsi que leurs sels physiologiquement compatibles, pour la fabrication d'un médicament pour le traitement du syndrome métabolique.

2. Utilisation des composés de formule I selon la revendication 1, **caractérisée en ce que** dans, celle-ci,
R1 signifie H, OH, COOH, alkyle en (C₁-C₈), alcényle en (C₂-C₈), aryle, un ou plusieurs groupes -CH₂ ou -CH-individuels dans les radicaux alkyle en (C₁-C₈), alcényle en (C₂-C₈) pouvant être remplacés par -O- et les radicaux alkyle, alcényle et aryle pouvant être substitués une ou plusieurs fois avec
F, Cl, Br, I, CF₃, NO₂, N₃, CN, =O, COOH, COO-alkyle en (C₁-C₆), CONH₂, CONH-alkyle en (C₁-C₆), CON[alkyle en (C₁-C₆)]₂, cycloalkyle, alkyle en (C₁-C₁₀), alcényle en (C₂-C₆), alcynyle en (C₂-C₆), O-alkyle en (C₁-C₆), O-CO-alkyle en (C₁-C₆), O-CO-aryle en (C₁-C₆), O-CO-hétérocycle en (C₁-C₆); PO₃H₂, P(O) (O-alkyle)₂, alkylène en (C₁-C₆)-P(O) (O-alkyle)₂, O-P(O)(OH)₂, O-P(O)(O-alkyle)₂, SO₃H, SO₂-NH₂, SO₂NH-alkyle en (C₁-C₆), SO₂N-[alkyle en (C₁-C₆)]₂, S-alkyle en (C₁-C₆), S-(CH₂)ₙ-aryle, S-(CH₂)ₙ-hétérocyclyle, SO-alkyle en (C₁-C₆), SO-(CH₂)ₙ-aryle, SO-(CH₂)ₙ-hétérocyclyle, SO₂-alkyle en (C₁-C₆), SO₂-(CH₂)ₙ-aryle, SO₂-(CH₂)ₙ-hétérocyclyle, SO₂-NH(CH₂)ₙ-aryle, SO₂-NH(CH₂)ₙ-hétérocyclyle, SO₂-N (alkyle en (C₁-C₆))(CH₂)ₙ-aryle, SO₂-N (alkyle en (C₁-C₆)) (CH₂)ₙ-hétérocyclyle, SO₂-N((CH₂)ₙ-aryle)₂, SO₂-N((CH₂)ₙ-(hétérocyclyle)₂, n pouvant être 0 à 6 et le radical aryle ou le radical hétérocyclique pouvant être substitué jusqu'à deux fois avec F, Cl, Br, OH, CF₃, SF₅, NO₂, CN, OCF₃, O-alkyle en (C₁-C₆), alkyle en (C₁-C₆), NH₂;
C(NH)(NH₂), NH₂, NH-alkyle en (C₁-C₆), N(alkyle en (C₁-C₆))₂, NH-acyle en (C₁-C₇), NH-CO-alkyle en (C₁-C₆), NH-COO-alkyle en (C₁-C₆), NH-CO-aryle, NH-CO-hétérocyclyle, NH-COO-aryle, NH-COO-hétérocyclyle, NH-CO-NH-alkyle en (C₁-C₆)), NH-CO-NH-aryle, NH-CO-NH-hétérocyclyle, N[alkyle en (C₁-C₆)]-CO-alkyle en (C₁-C₆), N [alkyle en (C₁-C₆)]-COO-alkyle en (C₁-C₆), N[alkyle en (C₁-C₆)]-CO-aryle, N[alkyle en (C₁-C₆)]-CO-hétérocyclyle, N[alkyle en (C₁-C₆)]-COO-aryle, N[alkyle en (C₁-C₆)]-COO-hétérocyclyle, N[alkyle en (C₁-C₆)]-CO-NH-alkyle en (C₁-C₆)), N [alkyle en (C₁-C₆)]-CO-NH-aryle, N [alkyle en (C₁-C₆)]-CO-NH-hétérocyclyle, N [alkyle en (C₁-C₆)]-CO-N (alkyle en (C₁-C₆))₂, N[alkyle en (C₁-C₆)]-CO-N(alkyle en (C₁-C₆))-aryle, N[alkyle en (C₁-C₆)]-CO-N(alkyle en (C₁-C₆))-hétérocyclyle, N[alkyle en (C₁-C₆)]-CON(aryle)₂, N [alkyle en (C₁-C₆)]-CON(hétérocyclyle)₂, N(aryle)-CO-alkyle en (C₁-C₆), N(hétérocyclyle)-CO-alkyle en (C₁-C₆), N(aryle)-COO-alkyle en (C₁-C₆), N(hétérocyclyle)-COO-alkyle en (C₁-C₆), N(aryle)-CO-aryle, N(hétérocyclyle)-CO-aryle, N(aryle)-COO-aryle, N(hétérocyclyle)-COO-aryle, N(aryle)-CO-NH-alkyle en (C₁-C₆), N(hétérocyclyle)-CO-NH-alkyle en (C₁-C₆), N(aryle)-CO-NH-aryle, N(hétérocyclyle)-CO-NH-aryle, N(aryle)-CO-N(alkyle en (C₁-C₆))₂, N(hétérocyclyle)-CO-N(alkyle en (C₁-C₆))₂, N(aryle)-CO-N[alkyle en (C₁-C₆)]-aryle, N(hétérocyclyle)-CO-N[alkyle en (C₁-C₆)]-aryle, N(aryle)-CO-N(aryle)₂, N(hétérocyclyle)-CON (aryle)₂, aryle, 0- (CH₂)ₙ-aryle, O- (CH₂)ₙ-hétérocyclyle, n pouvant être 0 à 6, le radical aryle ou hétérocyclyle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆, N (alkyle en (C₁-C₆))₂, SF₅, SO₂-CH₃, COOH, COO-alkyle en (C₁-C₆), CONH₂ ;
R2 signifie H, OH, COOH, alkyle en (C₁-C₈), cycloalkyle en (C₃-C₈), alcényle en (C₂-C₈), un ou plusieurs groupes -CH₂ ou -CH- individuels dans les radicaux alkyle en (C₁-C₈) ou alcényle en (C₂-C₈) pouvant être remplacés par -O- et les radicaux alkyle, cycloalkyle et alcényle pouvant être substitués une ou plusieurs fois avec
F, Cl, Br, I, CF₃, NO₂, N₃, CN, =O, COOH, COO-alkyle en (C₁-C₆), CONH₂, CONH-alkyle en (C₁-C₆), CON[alkyle en (C₁-C₆)]₂, cycloalkyle, alkyle en (C₁-C₁₀), alcényle en (C₂-C₆), alcynyle en (C₂-C₆), 0-alkyle en (C₁-C₆), O-CO-alkyle en (C₁-C₆), O-CO-aryle en (C₁-C₆), O-CO-hétérocycle en (C₁-C₆); PO₃H₂, P(O) (O-alkyle)₂, alkylène en (C₁-C₆)-P(O) (O-alkyle)₂, O-P(O)(OH)₂, O-P(O)(O-alkyle)2, SO₃H, SO₂-NH₂, SO₂NH-alkyle en (C₁-C₆), SO₂N-[alkyle en (C₁-C₆)]₂, S-alkyle en (C₁-C₆), S-(CH₂)ₙ-aryle, S-(CH₂)ₙ-hétérocyclyle, SO-alkyle en (C₁-C₆), SO-(CH₂₎ₙ-aryle, SO- (CH₂)ₙ-hétérocyclyle, SO₂-alkyle en (C₁-C₆), SO₂-(CH₂)ₙ-aryle, SO₂-(CH₂)ₙ-hétérocyclyle, SO₂-NH(CH₂)ₙ-aryle, SO₂-NH (CH₂)ₙ-hétérocyclyle, SO₂-N (alkyle en (C₁-C₆)) (CH2) n-aryle, SO₂-N (alkyle en (C₁-C₆)) (CH₂)ₙ-hétérocyclyle, SO₂-N((CH₂)ₙ-aryle)₂, SO₂-N((CH₂)ₙ-(hétérocyclyle)₂, n pouvant être 0 à 6 et le radical aryle ou le radical hétérocyclique pouvant être substitué jusqu'à deux fois avec F, Cl, Br, OH, CF₃, SF₅, NO₂, CN, OCF₃, O-alkyle en (C₁-C₆), alkyle en (C₁-C₆), NH₂ ;
C(NH)(NH₂), NH₂, NH-alkyle en (C₁-C₆), N (alkyle en (C₁-C₆))₂, NH-acyle en (C₁-C₇), NH-CO-alkyle en (C₁-C₆), NH-COO-alkyle en (C₁-C₆), NH-CO-aryle, NH-CO-hétérocyclyle, NH-COO-aryle, NH-COO-hétérocyclyle, NH-CO-NH-alkyle en (C₁-C₆)), NH-CO-NH-aryle, NH-CO-NH-hétérocyclyle, N[alkyle en (C₁-C₆)]-CO-alkyle en (C₁-C₆), N [alkyle en (C₁-C₆)]-COO-alkyle en (C₁-C₆), N[alkyle en (C₁-C₆)]-CO-aryle, N[alkyle en (C₁-C₆)]-CO-hétérocyclyle, N[alkyle en (C₁-C₆)]-COO-aryle, N[alkyle en (C₁-C₆)]-COO-hétérocyclyle, N[alkyle en (C₁-C₆)]-CO-NH-alkyle en (C₁-C₆)), N[alkyle en (C₁-C₆)]-CO-NH-aryle, N[alkyle en (C₁-C₆)]-CO-NH-hétérocyclyle, N[alkyle en (C₁-C₆)]-CO-N(alkyle en (C₁-C₆))₂, N[alkyle en (C₁-C₆)]-CO-N(alkyle en (C₁-C₆))-aryle, N[alkyle en (C₁-C₆)]-CO-N(alkyle en (C₁-C₆)) -hétérocyclyle, N[alkyle en (C₁-C₆)]-CON(aryle)₂, N[alkyle en (C₁-C₆)]-CON(hétérocyclyle)₂, N(aryle)-CO-alkyle en (C₁-C₆), N(hétérocyclyle)-CO-alkyle en (C₁-C₆), N(aryle)-COO-alkyle en (C₁-C₆), N(hétérocyclyle)-COO-alkyle en (C₁-C₆), N(aryle)-CO-aryle, N(hétérocyclyle)-CO-aryle, N(aryle)-COO-aryle, N(hétérocyclyle)-COO-aryle, N(aryle)-CO-NH-alkyle en (C₁-C₆), N(hétérocyclyle)-CO-NH-alkyle en (C₁-C₆), N(aryle)-CO-NH-aryle, N(hétérocyclyle)-CO-NH-aryle, N(aryle)-CO-N(alkyle en (C₁-C₆))₂, N(hétérocyclyle)-CO-N(alkyle en (C₁-C₆))₂, N(aryle)-CO-N[alkyle en (C₁-C₆)]-aryle, N(hétérocyclyle)-CO-N[alkyle en (C₁-C₆)]-aryle, N(aryle)-CO-N(aryle)₂, N(hétérocyclyle)-CON(aryle)₂, aryle, O-(CH₂)ₙ-aryle, O- (CH₂)ₙ-hétérocyclyle, n pouvant être 0 à 6, le radical aryle ou hétérocyclyle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH₂;
ou R1 et R2 forment ensemble un cycle aryle, cycloalkyle ou hétérocyclyle de 3 à 8 éléments, le cycle aryle, cycloalkyle ou hétérocyclyle pouvant être substitué avec F, Cl, Br, I, OH, CF₃, NO₂, CN, OCF₃, 0-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH ;
ainsi que leurs sels physiologiquement compatibles, pour la fabrication d'un médicament pour le traitement du syndrome métabolique.

3. Utilisation des composés de formule I selon la revendication 1, **caractérisée en ce que** dans, celle-ci,
R1 signifie H, OH, COOH, alkyle en (C₁-C₈), alcényle en (C₂-C₈), aryle, un ou plusieurs groupes -CH₂ ou -CH-individuels dans les radicaux alkyle en (C₁-C₈), alcényle en (C₂-C₈) pouvant être remplacés par -O- et les radicaux alkyle, alcényle et aryle pouvant être substitués une ou plusieurs fois avec
=O, aryle, le radical aryle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH₂ ;
R2 signifie H, alkyle en (C₁-C₈), alcényle en (C₂-C₈); ainsi que leurs sels physiologiquement compatibles, pour la fabrication d'un médicament pour le traitement du syndrome métabolique.

4. Utilisation des composés de formule I selon la revendication 1, **caractérisée en ce que**, dans celle-ci,
R1 signifie H, OH, alkyle en (C₁-C₈), alcényle en (C₂-C₄), un ou plusieurs groupes -CH₂ ou -CH- individuels dans les radicaux alkyle en (C₁-C₈), alcényle en (C₂-C₄) pouvant être remplacés par -O- et les radicaux alkyle et alcényle pouvant être substitués une ou plusieurs fois avec
=O, aryle, le radical aryle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH ;
R2 signifie H, alkyle en (C₁-C₈), alcényle en (C₂-C₄) ; ainsi que leurs sels physiologiquement compatibles, pour la fabrication d'un médicament pour le traitement du syndrome métabolique.

5. Utilisation des composés de formule I selon l'une quelconque des revendications 1 à 4, ainsi que de leurs sels physiologiquement compatibles pour la fabrication d'un médicament pour la réduction des lipides dans le plasma.

6. Utilisation des composés de formule I selon l'une quelconque des revendications 1 à 4, ainsi que de leurs sels physiologiquement compatibles pour la fabrication d'un médicament pour la réduction des acides gras libres (FFA) dans le plasma.

7. Utilisation des composés de formule I selon l'une quelconque des revendications 1 à 4, ainsi que de leurs sels physiologiquement compatibles pour la fabrication d'un médicament pour la réduction du glycérol dans le plasma.

8. Utilisation des composés de formule I selon l'une quelconque des revendications 1 à 4, ainsi que de leurs sels physiologiquement compatibles pour la fabrication d'un médicament pour la réduction des triglycérides dans le plasma.

9. Utilisation des composés de formule I selon l'une quelconque des revendications 1 à 4, ainsi que de leurs sels physiologiquement compatibles pour la fabrication d'un médicament pour la prophylaxie du diabète de type 2.

10. Utilisation des composés de formule I selon l'une quelconque des revendications 1 à 4, ainsi que de leurs sels physiologiquement compatibles pour la fabrication d'un médicament pour le traitement de la dyslipidémie diabétique.

11. Utilisation des composés de formule I selon l'une quelconque des revendications 1 à 4, ainsi que de leurs sels physiologiquement compatibles pour la fabrication d'un médicament pour le traitement de l'obésité.

12. Utilisation des composés de formule I selon l'une quelconque des revendications 1 à 4, dans laquelle R1 et R2 sont l'hydrogène, en tant que médicament.

13. Composés de formule I 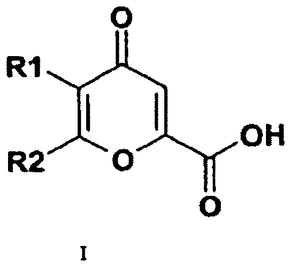 dans laquelle
R1 signifie alkyle en (C₁-C₈), alcényle en (C₂-C₄), les radicaux alkyle et alcényle pouvant être substitués une ou plusieurs fois avec F,
R2 signifie H ;
ainsi que leurs sels physiologiquement compatibles.

14. Composés de formule I selon la revendication 13, **caractérisés en ce que**, dans celle-ci,
R1 signifie alkyle en (C₂-C₈), le radical alkyle pouvant être substitué une ou plusieurs fois avec F,
R2 signifie H ;
ainsi que leurs sels physiologiquement compatibles.

15. Utilisation des composés de formule I selon la revendication 1, **caractérisés en ce que**, dans celle-ci,
R1 signifie H, OH, COOH, alkyle en (C₁-C₈), alcényle en (C₂-C₈), aryle, un ou plusieurs groupes -CH₂ ou -CH-individuels dans les radicaux alkyle en (C₁-C₈), alcényle en (C₂-C₈) pouvant être remplacés par -O- et les radicaux alkyle, alcényle et aryle pouvant être substitués une ou plusieurs fois avec
F, =O, aryle, le radical aryle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH₂ ;
R2 signifie H, alkyle en (C₁-C₈), alcényle en (C₂-C₈; ainsi que leurs sels physiologiquement compatibles, pour la fabrication d'un médicament pour le traitement du syndrome métabolique.

16. Utilisation des composés de formule I selon la revendication 1, **caractérisés en ce que**, dans celle-ci, R1 signifie H, OH, alkyle en (C₁-C₈), alcényle en (C₂-C₄), un ou plusieurs groupes -CH₂ ou -CH- individuels dans les radicaux alkyle en (C₁-C₈), alcényle en (C₂-C₄) pouvant être remplacés par -O- et les radicaux alkyle et alcényle pouvant être substitués une ou plusieurs fois avec
F, =O, aryle, le radical aryle pouvant être substitué une à 3 fois avec F, Cl, Br, I, OH, CF₃, NO₂, CN, OCF₃, O-alkyle en (C₁-C₆), alkyle en (C₁-C₆), NH₂, NH-alkyle en (C₁-C₆), N (alkyle en (C₁-C₆))₂, SF₅, SO₂-CH₃, COOH, COO-alkyle en (C₁-C₆), CONH₂ ;
R2 signifie H, alkyle en (C₁-C₈), alcényle en (C₂-C₄) ; ainsi que leurs sels physiologiquement compatibles, pour la fabrication d'un médicament pour le traitement du syndrome métabolique.
